# EUROPEAN PATENT SPECIFICATION

(11) **EP 1 935 916 B1**
(45) Date of publication and mention of the grant of the patent: **23.05.2012**
(21) Application number: 06797883.3
(22) Date of filing: 06.09.2006
(51) Int. Cl.: B01D 71/76, B01D 71/80, C07C 309/73, C07C 311/16, C08G 61/00, C08G 61/10

(54) **POLYARYLENE AND PROCESS FOR PRODUCING THE SAME**
POLYARYLEN UND HERSTELLUNGSVERFAHREN DAFÜR
POLYARYLÈNE ET SON PROCÉDÉ DE PRODUCTION

(30) Priority: 13.10.2005 JP 2005298604; 13.10.2005 JP 2005298605; 16.03.2006 JP 2006072576; 23.03.2006 JP 2006080248
(43) Date of publication of application: 25.06.2008
(73) Proprietor: Sumitomo Chemical Company, Limited, Tokyo 104-8260 (JP)
(72) Inventor: HIDA, Noriyuki, Sakai-shi, Osaka 5900022 (JP); ODA, Seiji, Ibaraki-shi, Osaka 5670841 (JP); ONODERA, Toru, Tsukuba-shi, Ibaraki 3003261 (JP); KAMIKAWA, Takashi, Nara 6308442 (JP)
(74) Representative: Vossius & Partner
(86) International application number: PCT/JP2006/318091
(87) International publication number: WO 2007/043274

(56) References cited:
- EP-A- 0 841 340
- EP-A- 0 905 202
- WO-A1-97/34903
- WO-A1-98/34988
- WO-A1-2005/075535
- BE-A1- 615 985
- JP-A- 2003 238 665
- JP-A- 2005 220 193
- JENNIFER A. IRVIN ET AL.: "Synthesis and Characterization of Chiral Conjugated Polymers for Optical Waveguides" OSA TRENDS IN OPTICS AND PHOTONICS, vol. 64, 2002, pages 88-92, XP009122682 US
- KLAVETTER F.L.: 'PHOTOLUMINESCENCE AND ELECTROLUMINESCENCE OF TRANSPARENT POLYPHENYLENE DERIVATIVES' POLYM. MATER. SCI. ENG. vol. 69, 1993, pages 153 - 154, XP009122664
- P. R. ADAMS AND W. J. HICKINBOTTOM: "756. Alkylation of the aromatic nucleus. Part XIII. p-Xylene", JOURNAL OF THE CHEMICAL SOCIETY, 1 January 1965 (1965-01-01), pages 4097-4100, DOI: 10.1039/JR9650004097

## Description

### Technical Field

The present invention relates to a polyarylene and a method for producing the same.

### Background Art

A polyarylene having sulfonic acid groups is useful as a polyelectrolyte for proton-exchange membrane fuel cell. As method for producing it, a method using benzene as a monomer (e.g. US patent No. 3,376,235), a method using a dihalobenzenesulfonate as a monomer (e.g. JP 2003-238665 A and W02005/075535) and a method using phenyl dibromobenzenesulfonate and phenyl boric acid as monomers (e.g. Macromol. Rapid. Commun., 15, 669-676 (1994)) have been known.

### Disclosure of the Invention

The present disclosure provides a dihalobenzene compound represented by the formula (1): 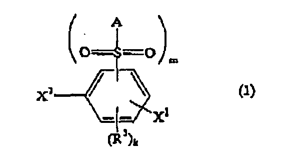 wherein A represents an amino group substituted with one or two hydrocarbon groups wherein the sum of number of carbon atoms of the hydrocarbon group or groups is 3 to 20, or a C3-C20 alkoxy group, and the above-mentioned hydrocarbon group and the C3-C20 alkoxy group may be substituted with at least one group selected from the group consisting of a fluorine atom, a C1-C20 alkoxy group, a C6-C20 aryl group, a C6-C20 aryloxy group, a C2-C20 acyl group and a cyano group,
R¹ represents a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the C1-C20 alkyl group, the C1-C20 alkoxy group, the C6-C20 aryl group, the C6-C20 aryloxy group and the C2-C20 acyl group may be substituted with at least one substituent selected from the group consisting of a fluorine atom, a cyano group, a C1-C20 alkoxy group, a C6-C20 aryl group and a C6-C20 aryloxy group, and when multiple R¹s exist, R¹s may be the same groups or different groups, and the neighboring two R¹s may be bonded to form a ring,
X¹ represents a chlorine atom, a bromine atom or an iodine atom, m represents 1 or 2, and k represents 4-mₒ

The present invention discloses a polyarylene comprising a repeating unit represented by the formula (2): 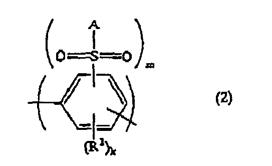 wherein A, R¹, m and k represent the meanings as defined in the appended claims,
a method for producing the above-mentioned polyarylene,
a method for producing a polyarylene comprising a repeating unit represented by the formula (7): 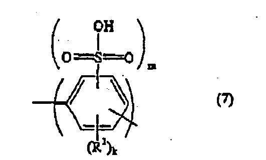 wherein R¹, m and k represent the same meanings as defined above, from the above-mentioned polyarylene.
A method for producing the above-mentioned dihalobenzene compound represented by the formula (1) is disclosed, as well.

### Best Mode for Carrying Out the Present Invention

First, a dihalobenzene compound represented by the formula (1): 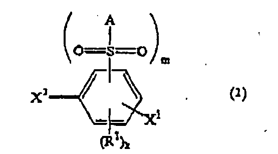 (hereinafter, simply referred to as the dihalobenzene compound (1)) will be illustrated.

A represents a C3-C20 alkoxy group.

Examples of the C3-C20 alkoxy group include a linear, branched chain or cyclic C3-C20 alkoxy group such as a n-propoxy, isopropoxy, n-butoxy, sec-butoxy, tert-butoxy, n-pentyloxy, 2,2-methylpropoxy, n-hexyloxy, cyclohexyloxy, n-heptyloxy, n-octyloxy, n-nonyloxy, n-decyloxy, n-undecyloxy, n-dodecyloxy, n-tridecyloxy, n-tetradecyloxy, n-pentadecyloxy, n-hexadecyloxy, n-heptadecyloxy, n-octadecyloxy, n-nonadecyloxy and n-icosyloxy group, and the isobutoxy group, the 2,2-dimethypropoxy group and cyclohexyloxy group are preferable.

The above-mentioned C3-C20 alkoxy group may be substituted with at least one group selected from the group consisting of a fluorine atom, a C1-C20 alkoxy group, a C6-C20 aryl group, a C6-C20 aryloxy group, a C2-C20 acyl group and a cyano group.

Examples of the C1-C20 alkoxy group include a linear, branched chain or cyclic C1-C20 alkoxy group such as a methoxy, ethoxy, n-propoxy, isopropoxy, n-butoxy, isobutoxy, sec-butoxy, tert-butoxy, n-pentyloxy, 2,2-methylpropoxy, cyclopentyloxy, n-hexyloxy, cyclohexyloxy, n-heptyloxy, 2-methylpentyloxy, n-octyloxy, 2-ethylhexyloxy, n-nonyloxy, n-decyloxy, n-undecyloxy, n-dodecyloxy, n-tridecyloxy, n-tetradecyloxy, n-pentadecyloxy, n-hexadecyloxy, n-heptadecyloxy, n-octadecyloxy, n-nonadecyloxy and n-icosyloxy group.

Examples of the C6-C20 aryl group include a phenyl, 1-naphthyl, 2-naphthyl, 3-phenanthryl and 2-anthryl group. Examples of the C6-C20 aryloxy group include those composed of the above-mentioned C6-C20 aryl group and an oxygen atom such as a phenoxy, 1-naphthyloxy, 2-naphthyloxy, 3-phenanthryloxy and 2-anthryloxy group.

Examples of the C2-C20 acyl group include a C2-C20 aliphatic or aromatic acyl group such as an acetyl, propionyl, butyryl, isobutyryl, benzoyl, 1-naphthoyl and 2-naphthoyl group.

Among them, a C3-C20 unsubstituted alkoxy group is preferable as A, and the isobutoxy group, the 2,2-dimethylpropoxy group and the cyclohexyloxy group are more preferable.

R¹ represents a hydrogen atom, a fluorine atom, a C1-C20 alkyl group, a C1-C20 alkoxy group, a C6-C20 aryl group, a C6-C20 aryloxy group, a C2-C20 acyl group or a cyano group.

Examples of the C1-C20 alkyl group include a linear, branched chain or cyclic C1-C20 alkyl group such as a methyl, ethyl, n-propyl, isopropyl, n-butyl, isobutyl, sec-butyl, tert-butyl, n-pentyl, 2,2-methylpropyl, cyclopentyl, n-hexyl, cyclohexyl, n-heptyl, 2-methylpentyl, n-octyl, 2-ethylhexyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl and n-icosyl group.

Examples of the C1-C20 alkoxy group, the C6-C20 aryl group, the C6-C20 aryloxy group and the C2-C20 acyl group include those as same as described above.

The C1-C20 alkyl group, the C1-C20 alkoxy group, the C6-C20 aryl group, the C6-C20 aryloxy group and the C2-C20 acyl group may be substituted with at least one substituent selected from the group consisting of a fluorine atom, a cyano group, a C1-C20 alkoxy group, a C6-C20 aryl group and a C6-C20 aryloxy group, and examples of the C1-C20 alkoxy group, the C6-C20 aryl group and the C6-C20 aryloxy group include those as same as described above.

When multiple R¹s exist, R¹s may be the same groups or different groups, and the neighboring two R¹s may be bonded to form a ring.

Among them, the hydrogen atom is preferable as R¹.

X¹ represents a chlorine atom, a bromine atom or an iodine atom, and the chlorine atom and the bromine atom are preferable, and m represents 1 or 2, and k represents 4-m, and m preferably represents 1.

Examples of the dihalobenzene compound (1) include isopropyl 2,5-dichlorobenzenesulfonate, isobutyl 2,5-dichlorobenzenesulfonate, 2,2-dimethylpropyl 2,5-dichlorobenzenesulfonate, cyclohexyl 2,5-dichlorobenzenesulfonate, n-octyl 2,5-dichlorobenzenesulfonate, n-pentadecyl
2,5-dichlorobenzenesulfonate, n-icosyl 2,5-diachlorobenzenesulfonate, isopropyl 3,5-dichlorobenzenesulfonate, isobutyl 3,5-dichlorobenzenesulfonate, 2,2-dimethylpropyl 3,5-dichlorobenzenesulfonate, cyclohexyl 3,5-dichlorobenzenesulfonate, n-octyl 3,5-dichlorobenzenesulfonate, n-pentadecyl 3,5-dichlorobenzenesulfonate, n-icosyl 3,5-dichlorobenzenesulfonate, N,N-diethyl-3,5-dichlorobenzenesulfonamide, N,N-diisopropyl-3,5-dichlorobenzenesulfonomide, N-(2,2-dimethylpropyl)-3,5-dichlorobenzenesulfonamide, N-n-dodecyl-3,5-dichlorobenzenesulfonamide, N-n-icosyl-3,5-dichlorobenzenesulfonamide, isopropyl 2,5-dibromobenzenesulfonate, isobutyl 2,5-dibromobenzenesulfonate, 2,2-dimethylpropyl 2,5-dibromobenzenesulfonate, cyclohexyl 2,5-dibromobenzenesulfonate, n-octyl 2,5-dibromobenzenesulfonate, n-pentadecyl 2,5-dibromobenzenesulfonate, n-icosyl 2,5-dibromobenzenesulfonate, isopropyl 3,5-dibromobenzenesulfonate, isobutyl 3,5-dibromobenzenesulfonate, 2,2-dimethylpropyl 3,5-dibromobenzenesulfonate, cyclohexyl 3,5-dibromobenzenesulfonate, n-octyl 3,5-dibromobenzenesulfonate, n-pentadecyl 3,5-dibromobenzenesulfonate, n-icosyl 3,5-dibromobenzenesulfonate, isopropyl 2,5-diiodobenzenesulfonate, isobutyl 2,5-diiodobenzenesulfonate, 2,2-dimethylpropyl 2,5-diiodobenzenesulfonate, cyclohexyl 2,5-diiodobenzenesulfonate, n-octyl 2,5-diiodobenzenesulfonate, n-pentadecyl 2,5-diiodobenzenesulfonate, n-icosyl 2,5-diiodobenzenesulfonate, isopropyl 3,5-diiodobenzenesulfonate, isobutyl 3,5-diiodobenzenesulfonate, 2,2-dimethylpropyl 3,5-diiodobenzenesulfonate, cyclohexyl 3,5-diiodobenzenesulfonate, n-octyl 3,5-diiodobenzenesulfonate, n-pentadecyl 3,5-diiodobenzenesulfonate, n-icosyl 3,5-diiodobenzenesulfonate, 2,2-dimethylpropyl 2,4-dichlorobenzenesulfonate, 2 ,2-dimethylpropyl 2,4-dibromobenzenesulfonate, 2,2-dimethylpropyl 2,4-diiodobenzenesulfonate, 2,2-dimethylpropyl 2,4-dichloro-5-methylbenzenesulfonate, 2,2-dimethylpropyl 2,5-dichloro-4-methylbenzenesulfonate, 2,2-dimethylpropyl 2,4-dibromo-5-methylbenzenesulfonate, 2,2-dimethylpropyl 2,5-dibromo-4-methylbenzenesulfonate, 2,2-dimethylpropyl 2,4-diiodo-5-methylbenzenesulfonate, 2,2-dimethylpropyl 2,5-diiodo-4-methylbenzenesulfonate, 2,2-dimethylpropyl 2,4-dichloro-5-methoxybenzenesulfonate, 2,2-dimethylpropyl 2,5-dichloro-4-methoxybenzenesulfonate, 2,2-dimethylpropyl 2,4-dibromo-5-methoxybenzenesulfonate, 2,2-dimethylpropyl 2,5-dibromo-4-methoxybenzenesulfonate, 2,2-dimethylpropyl 2,4-diiodo-5-methoxybenzenesulfonate, and 2,2-dimethylpropyl 2,5-diiodo-4-methoxybenzenesulfonate.

Among them, 2,2-dimethylpropyl 2,5-dichlorobenzenesulfonate, isobutyl 2,5-dichlorobenzenesulfonate, cyclohexyl 2,5-dichlorobenzenesulfonate, 2,2-dimethylpropyl 2,5-dibromobenzenesulfonate, isobutyl 2,5-dibromobenzenesulfonate, and cyclohexyl 2,5-dibromobenzenesulfonate are preferable.

A polyarylene can be produced by polymerizing a monomer composition comprising the dihalobenzene compound (1). A polyarylene can also be produced by polymerizing the dihalobenzene compound (1) only. The polyarylene and the method for producing the same will be illustrated below.

Specific examples of the polyarylene include a polyarylene comprising a repeating unit represented by the formula (2): 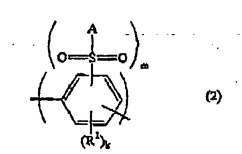 wherein A, R¹, m and k represents the same meanings as defined above (hereinafter, simply referred to as the repeating unit (2)), a polyarylene consisting of the above-mentioned repeating unit (2), a polyarylene comprising the above-mentioned repeating unit (2) and a segment represented by the formula (3): 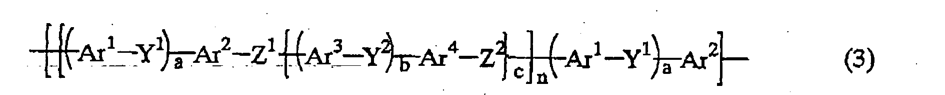 wherein a, b and c are the same or different, and each represents 0 or 1, and n represents a integer of 5 or more,
Ar¹, Ar³ and Ar⁴ are the same or different, and each represents a divalent aromatic group, and the divalent aromatic group may be substituted with at least one substituent selected from the group consisting of
a C1-C20 alkyl group which may be substituted with at least one selected from the group consisting of a fluorine atom, a cyano group, a C1-C20 alkoxy group, a C6-C20 aryl group and a C6-C20 aryloxy group,
a C1-C20 alkoxy group which may be substituted with at least one selected from the group consisting of a fluorine atom, a cyano group, a C1-C20 alkoxy group, a C6-C20 aryl group and a C6-C20 aryloxy group,
a C6-C20 aryl group which may be substituted with at least one selected from the group consisting of a fluorine atom, a cyano group, a C1-C20 alkoxy group and a C6-C20 aryloxy group,
a C6-C20 aryloxy group which may be substituted with at least one selected from the group consisting of a fluorine atom, a cyano group, a C1-C20 alkoxy group and a C6- C20 aryloxy group, and
a C2-C20 acyl group which may be substituted with at least one selected from the group consisting of a fluorine atom, a cyano group, a C1-C20 alkoxy group, a C6-C20 aryl group and a C6- C20 aryloxy group.
Y¹ and Y² are the same or different, and each represents a single bond, a carbonyl group, a sulfonyl group, 2,2-isopropylidene group, 2,2-hexafluoroisopropylidene group or a fluorene-9,9-diyl group, and
Z¹ and Z² are the same or different, and each represents an oxygen or sulfur atom (hereinafter, simply referred to as the segment (3)), and a polyarylene comprising the above-mentioned repeating unit (2) and a repeating unit represented by the formula (4):

-Ar⁵- (4)

wherein Ar⁵ represents a divalent aromatic group, and the divalent aromatic group may be substituted with at least one substituent selected from the group consisting of
a C1-C20 alkyl group which may be substituted with at least one selected from the group consisting of a fluorine atom, a cyano group, a C1-C20 alkoxy group, a C6-C20 aryl group and a C6-C20 aryloxy group,
a C1-C20 alkoxy group which may be substituted with at least one selected from the group consisting of a fluorine atom, a cyano group, a C1-C20 alkoxy group, a C6-C20 aryl group and a C6-C20 aryloxy group,
a C6-C20 aryl group which may be substituted with at least one selected from the group consisting of a fluorine atom, a cyano group, a C1-C20 alkoxy group and a C6-C20 aryloxy group,
a C6-C20 aryloxy group which may be substituted with at least one selected from the group consisting of a fluorine atom, a cyano group, a C1-C20 alkoxy group and a C6- C20 aryloxy group, and
a C2-C20 acyl group which may be substituted with at least one selected from the group consisting of a fluorine atom, a cyano group, a C1-C20 alkoxy group, a C6-C20 aryl group and a C6- C20 aryloxy group (hereinafter, simply referred to as the repeating unit (4)).

In the polyarylene comprising the repeating unit (2), at least two repeating unit (2) are usually continued.

The polyarylene comprising the repeating unit (2) may have a repeating unit or units other than the repeating unit (2) and a segment or segments. The polyarylene comprising the repeating unit (2) and the segment (3) may be a polyarylene consisting of the repeating unit (2) and the-segment (3), and may have a repeating unit or units and a segment or segments other than the repeating unit (2) and the segment (3) in addition to the repeating unit (2) and the segment (3). The polyarylene comprising the repeating units (2) and (4) may be a polyarylene consisting of the repeating units (2) and (4), and may have a repeating unit or units and a segment or segments other than the repeating units (2) and (4) in addition to the repeating units (2) and (4).

The weight average molecular weight of these polyarylenes in terms of polystyrene is usually 1,000 to 1,000,000.

Specific examples of the repeating unit (2) include repeating units represented by the following formulae (2a) to (2c) :

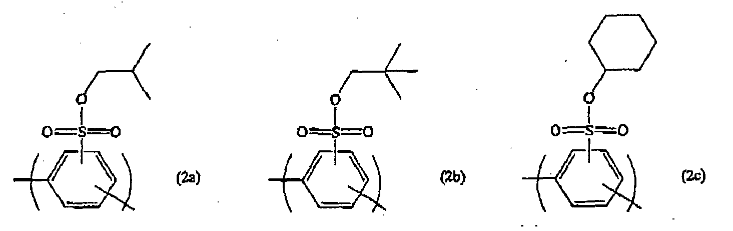

Examples of the divalent aromatic group in the segment (3) include a divalent monocyclic aromatic group such as a 1,3-phenylene and 1,4-phenylene group, a divalent condensed ring type aromatic group such as a naphthalene-1,3-diyl, naphthalene-1,4-diyl, naphthalene-1,5-diyl, naphthalene-1,6-diyl, naphthalene-1,7-diyl, naphthalene-2,6-diyl and naphthalene-2,7-diyl group, and a divalent heteroaromatic group such as a pyridine-2,5-diyl, pyridine-2,6-diyl, quinoxaline-2,6-diyl and thiophene-2,5-diyl group. Among them, the divalent monocyclic aromatic group and the divalent condensed ring type aromatic group are preferable, and the 1,4-phenylene group, the naphthalene-1,4-diyl group, the naphthalene-1,5-diyl group, the naphthalene-2,6-diyl group and the naphthalene-2,7-diyl group are more preferable.

The above-mentioned divalent aromatic group may be substituted with at least one substituent selected from the group consisting of a C1-C20 alkyl group which may be substituted with at least one selected from the group consisting of a fluorine atom, a cyano group, a C1-C20 alkoxy group, a C6-C20 aryl group and a C6-C20 aryloxy group; a C1-C20 alkoxy group which may be substituted with at least one selected from the group consisting of a fluorine atom, a cyano group, a C1-C20alkoxy group, a C6-C20 aryl group and a C6-C20 aryloxy group; a C6-C20 aryl group which may be substituted with at least one selected from the group consisting of a fluorine atom, a cyano group, a C1-C20 alkoxy group and a C6-C20 aryloxy group; a C6-C20 aryloxy group which may be substituted with at least one selected from the group consisting of a fluorine atom, a cyano group, a C1-C20 alkoxy group and a C6- C20 aryloxy group; and a C2-C20 acyl group which may be substituted with at least one selected from the group consisting of a fluorine atom, a cyano group, a C1-C20 alkoxy group, a C6-C20 aryl group and a C6- C20 aryloxy group.

Examples of the C1-C20 alkyl group, the C1-C20 alkoxy group, the C6-C20 aryl group, the C6-C20 aryloxy group and the C2-C20 acyl group include the same as described above.

Specific examples of the segment (3) include segments represented by the following formulae (3a) to (3y), and in the following formulae, n represents the same meaning as defined above, and n is preferably 5 or more, and more preferably 10 or more. The weight-average molecular weight of the segment (3) in terms of polystyrene is usually 2,000 or more, and preferably 3,000 or more.

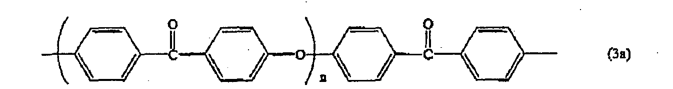

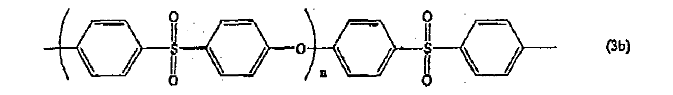

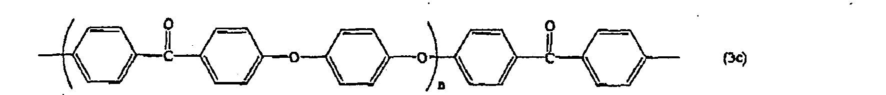

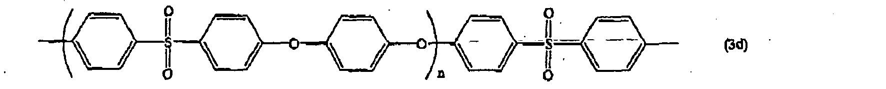

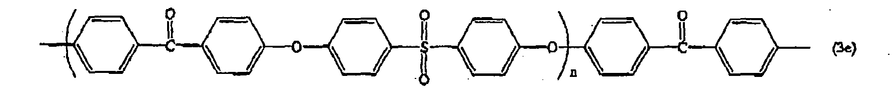

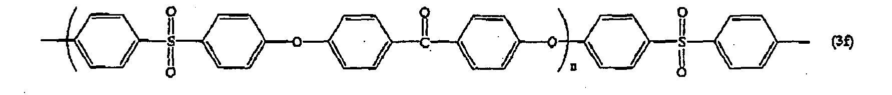

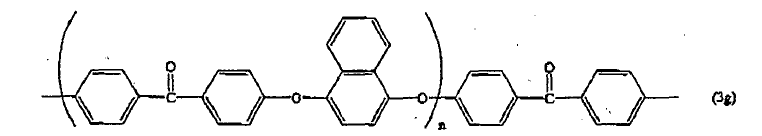

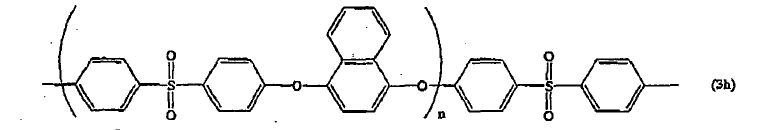

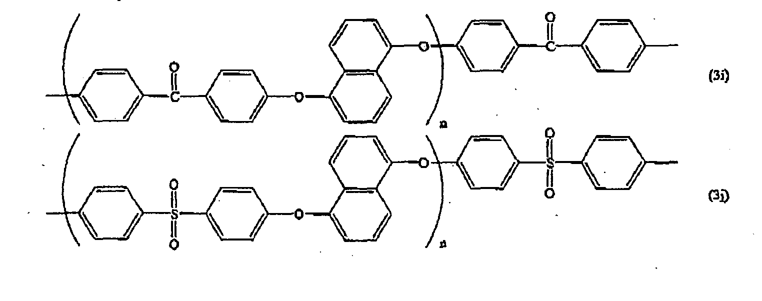

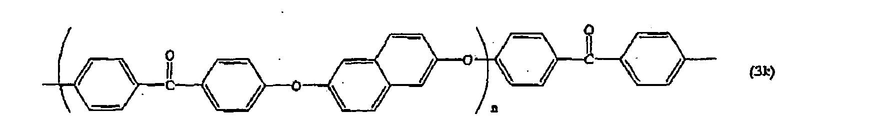

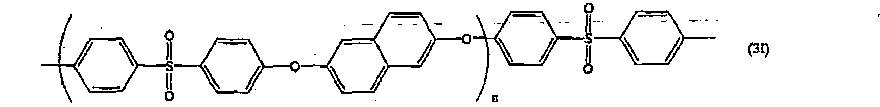

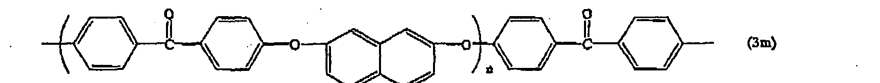

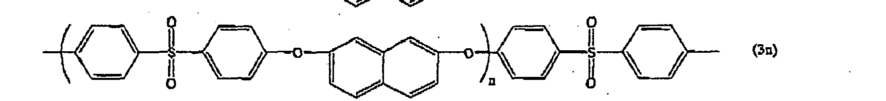

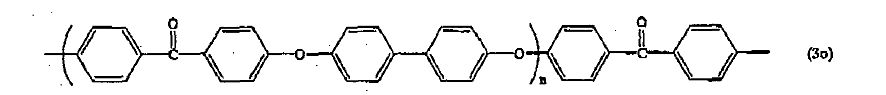

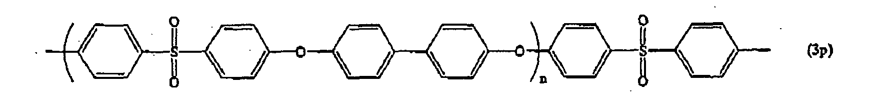

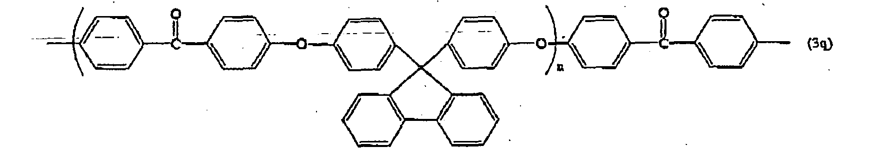

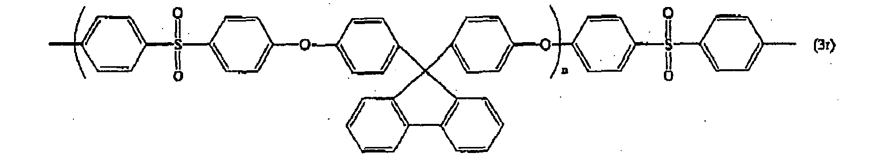

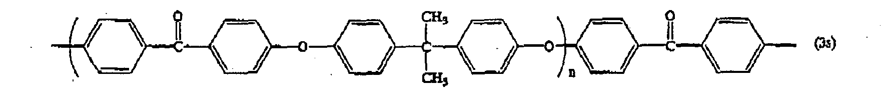

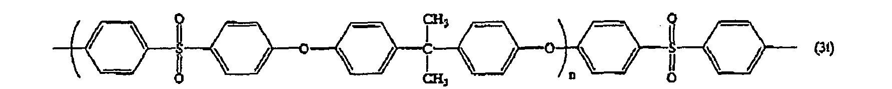

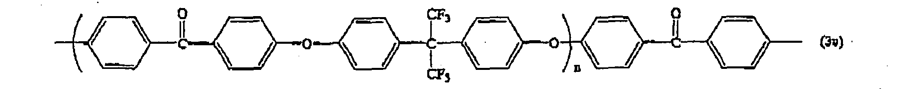

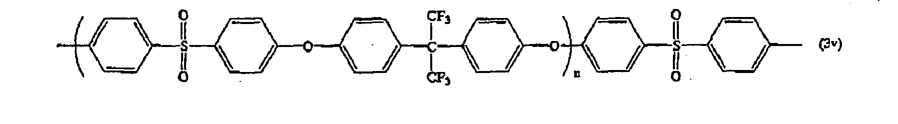

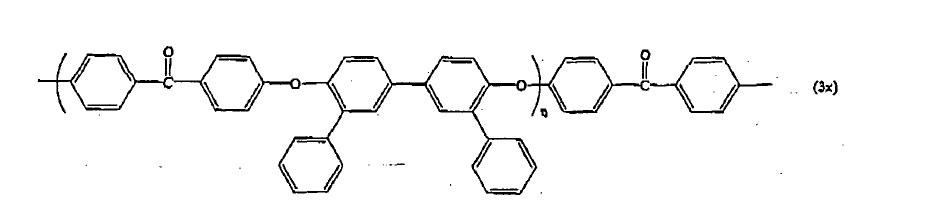

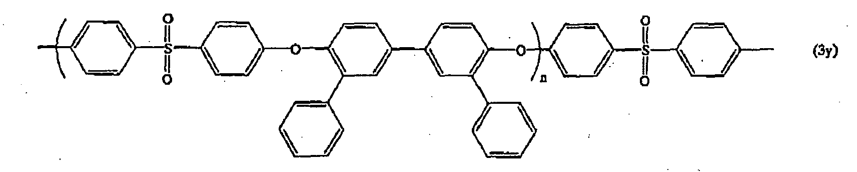

Examples of the polyarylene comprising the repeating unit (2) and the segment (3) include a polyarylene comprising any one of the above-mentioned repeating units represented by the formulae (2a) to (2c) and any one of the above-mentioned segments represented by the formulae (3a) to (3y). Specifically, polyarylenes represented by the following formulae (I) to (V) are exemplified. In the following formulae, n represents the same meaning as defined above and p represents an integer of 2 or more.

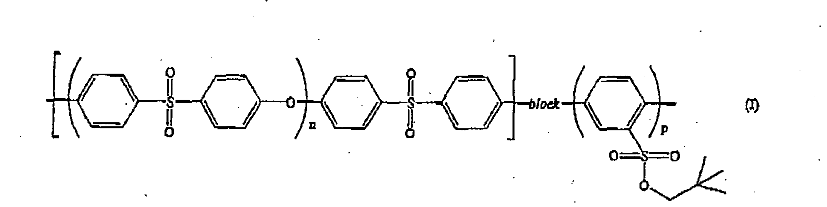

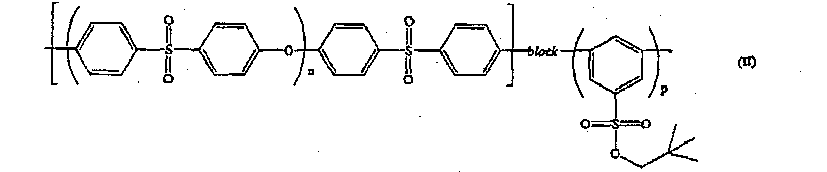

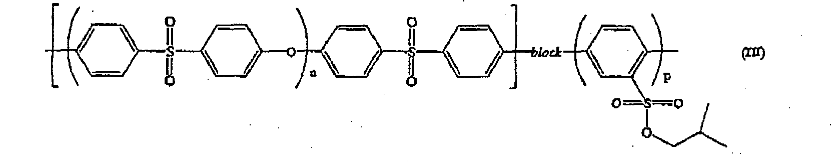

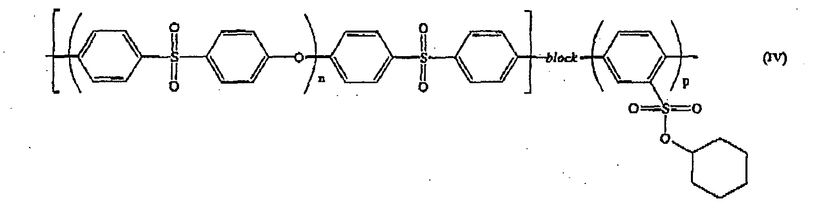

The amount of the repeating unit (2) in the polyarylene comprising the repeating unit (2) and the segment (3) is preferably 5% by weight or more and 95% by weight or less, and more preferably 30% by weight or more and 90% by weight or less. The amount of the segment (3) in the polyarylene comprising the repeating unit (2) and the segment (3) is preferably 5% by weight or more and 95% by weight or less, and more preferably 10% by weight or more and 70% by weight or less.

Examples of the divalent aromatic group in the repeating unit (4) include the same as the divalent aromatic group in the segment (3) described above. The divalent aromatic group may be substituted with at least one substituent selected from the group consisting of a C1-C20 alkyl group which may be substituted with at least one selected from the group consisting of a fluorine atom, a cyano group, a C1-C20 alkoxy group, a C6-C20 aryl group and a C6-C20 aryloxy group; a C1-C20 alkoxy group which may be substituted with at least one selected from the group consisting of a fluorine atom, a cyano group, a C1-C20 alkoxy group, a C6-C20 aryl group and a C6-C20 aryloxy group; a C6-C20 aryl group which may be substituted with at least one selected from the group consisting of a fluorine atom, a cyano group, a C1-C20 alkoxy group and a C6-C20 aryloxy group; a C6-C20 aryloxy group which may be substituted with at least one selected from the group consisting of a fluorine atom, a cyano group, a C1-C20 alkoxy group and a C6- C20 aryloxy group; and a C2-C20 acyl group which may be substituted with at least one selected from the group consisting of a fluorine atom, a cyano group, a C1-C20 alkoxy group, a C6-C20 aryl group and a C6- C20 aryloxy group. Examples of the C1-C20 alkyl group, the C1-C20 alkoxy group, the C6-C20 aryl group, the C6-C20 aryloxy group and the C2-C20 acyl group include the same as described above.

Specific examples of the repeating unit (4) include repeating units represented by the formulae (4a) and (4b).

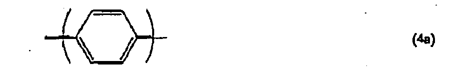

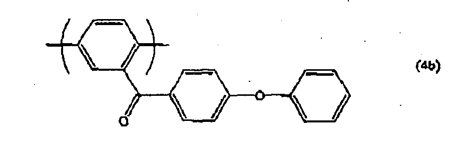

Examples of the polyarylene comprising the repeating unit (2) and the repeating unit (4) include polyarylenes comprising any one of the above-mentioned repeating units represented by the formulae (2a) to (2c) and any one of the above-mentioned repeating units represented by the formulae (4a) to (4b). Specifically, polyarylenes represented by the following formulae (VIII) to (X) are exemplified.

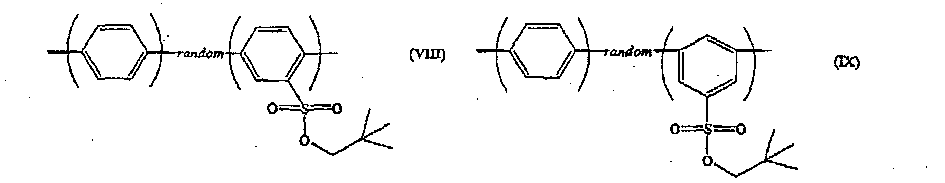

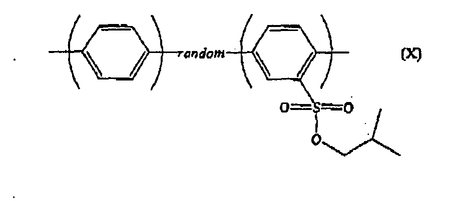

The amount of the repeating unit (2) in the polyarylene comprising the repeating unit (2) and the repeating unit (4) is preferably 5% by weight or more and 95% by weight or less, and more preferably 30% by weight or more and 90% by weight or less. The amount of the repeating unit (4) in the polyarylene comprising the repeating unit (2) and the repeating unit (4) is preferably 5% by weight or more and 95% by weight or less, and more preferably 10% by weight or more and 70% by weight or less.

The polyarylene comprising the repeating unit (2) can be produced by polymerizing a monomer composition comprising the dihalobenzene compound (1) in the presence of a nickel compound. The polyarylene consisting of the repeating unit (2) can be produced by polymerizing the dihalobenzene compound (1) only in the presence of a nickel compound. The polyarylene comprising the repeating unit (2) and the segment (3) can be produced by polymerizing a monomer composition comprising the dihalobenzene compound (1) and a compound represented by the formula (5): 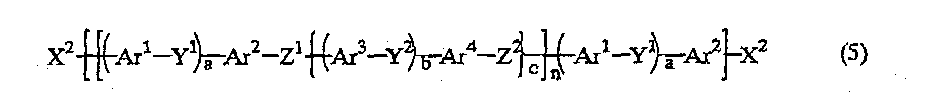 wherein a, b, c, n, Ar¹, Ar², Ar³, Ar⁴, Y¹, Y², Z¹ and Z² are the same meanings as defined above and X² represents a chlorine, bromine or iodine atom (hereinafter, simply referred to as the compound (5)), in the presence of a nickel compound. The polyarylene comprising the repeating unit (2) and the segment (3) can also be produced by polymerizing the dihalobenzene compound (1) only in the presence of a nickel compound and then further conducting a polymerization reaction by adding the compound (5).

The polyarylene comprising the repeating unit (2) and the repeating unit (4) can be produced by polymerizing a monomer composition comprising the dihalobenzene compound (1) and a compound represented by the formula (6):

X³-Ar⁵-X³ (6)

wherein Ar⁵ is the same meaning as defined above and X³ represents a chlorine, bromine or iodine atom (hereinafter, the compound (6)), in the presence of a nickel compound.

Examples of the compound (5) include the following compounds and the following compounds wherein the terminal chlorine atoms are substituted with bromine atoms.

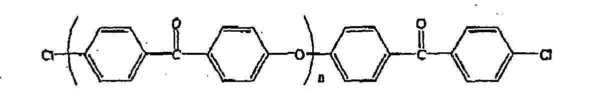

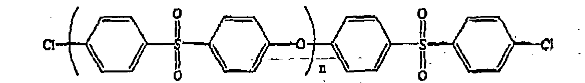

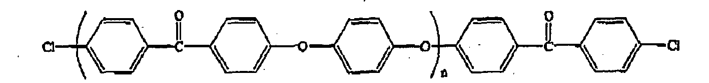

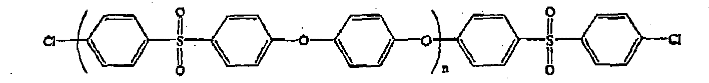

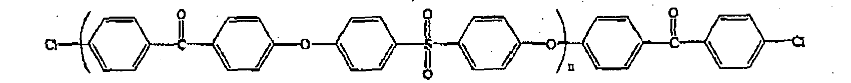

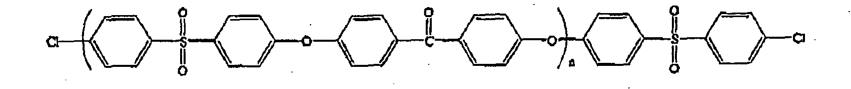

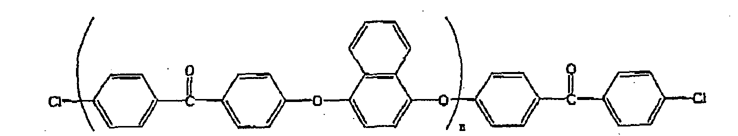

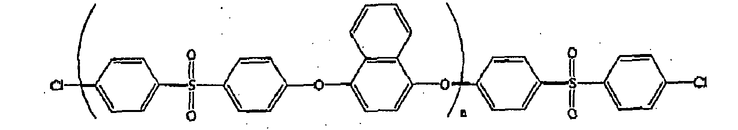

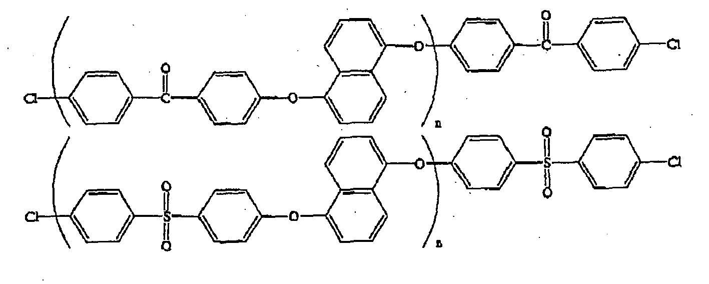

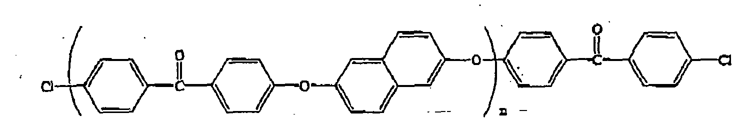

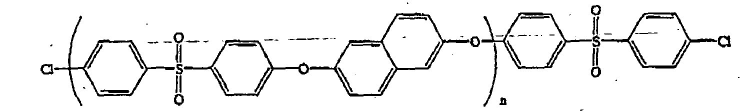

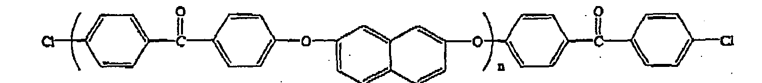

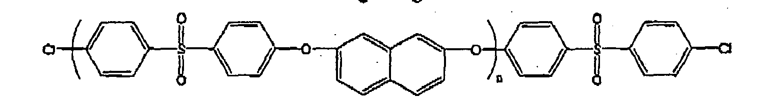

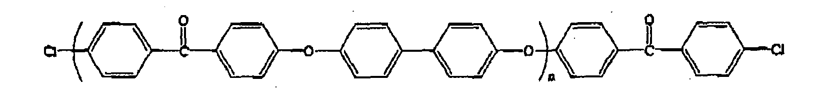

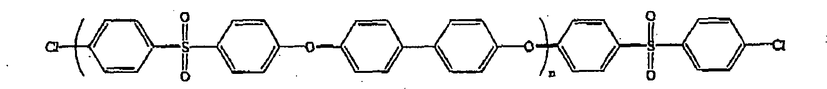

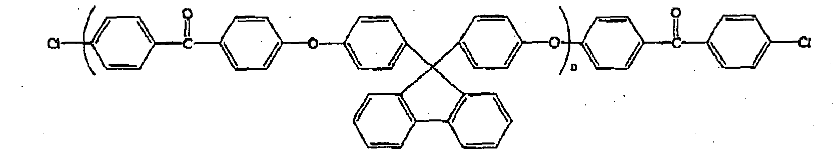

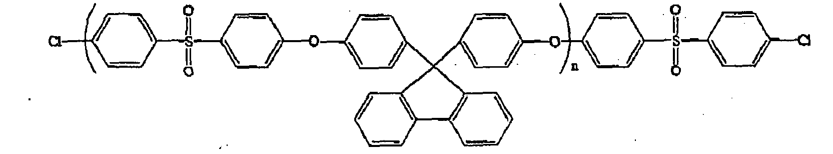

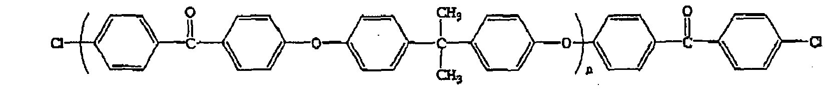

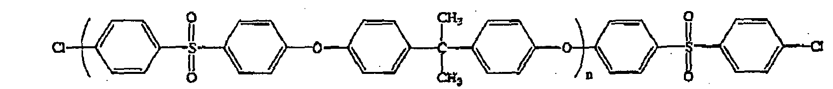

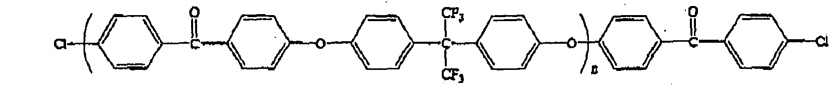

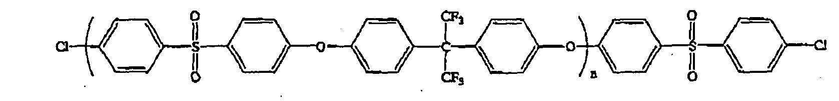

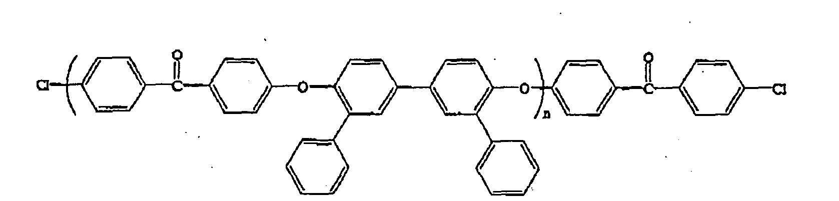

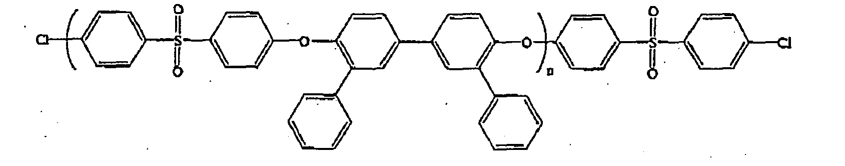

As the compound (5), one produced according to known methods such as JP Patent No. 2745727 may be used or a commercially available one may be used. Examples of the commercially available one include SUMIKA EXCEL PES manufactured by Sumitomo Chemical Company, Limited.

As the compound (5), one having 2,000 or more of weight-average molecular weight in terms of polystyrene is preferably used, and one having 3, 000 or more of weight-average molecular weight in terms of polystyrene is more preferably used.

Examples of the compound (6) include 1,3-dichlorobenzene, 1,4-dichlorobenzene,1,3-dibromobenzene, 1,4-dibromobenzene, 1,3-diiodobenzene, 1,4-diiodobenzene, 2,4-dichlorotoluene, 2,5-dichlorotoluene. 3,5-dichlorotoluene, 2,4-dibromotoluene, 2,5-dibromotoluene, 3,5-dibromotoluene, 2,4-diiodotoluene, 2,5-diiodotoluene, 3,5-diiodotoluene, 1,3-dichloro-4-methoxybenzene, 1,4-dichloro-3-methoxybenzene, 1,3-dibromo-4-methoxybenzene, 1,4-dibromo-3-methoxybenzene, 1,3-diiodo-4-methoxybenzene, 1,4-diiodo-3-methoxybenzene, 1,3-dichloro-4-acetoxybenzene, 1,4-dichloro-3-acetoxybenzene, 1,3-dibromo-4-acetoxybenzene, 1,4-dibromo-3-acetoxybenzene, 1,3-diiodo-4-acetoxybenzene, 1,4-diiodo-3-acetoxybenzene and 2,5-dichloro-4'-phenoxybenzophenone.

As the compound (6), a commercially available one is usually used.

The content of the repeating unit (2) in the polyarylene obtained can be adjusted by adjusting arbitrarily the content of the dihalobenzene compound (1) in the monomer composition.

Examples of the nickel compound include a zerovalent nickel compound such as bis(cyclooctadiene)nickel(0), (ethylene)bis(triphenylphosphine)nickel(0) and tetrakis(triphenylphosphine)nickel(0), and a divalent nickel compound such as a nickel halide (e.g. nickel fluoride, nickel chloride, nickel bromide, nickel iodide etc.), nickel carboxylate (e.g. nickel formate, nickel acetate etc.), nickel sulfate, nickel carbonate, nickel nitrate, nickel acetylacetonate and (dimethoxyethane)nickel chloride, and bis(cyclooctadiene)nickel(0) and the nickel halide are preferable.

When the amount of the nickel compound to be used is small, a polyarylene having a small molecular weight tends to be obtained, and when the amount thereof is high, a polyarylene having a large molecular weight tends to be obtained. Therefore, the amount of the nickel compound to be used may be decided depending on the desirable molecular weight of the polyarylene. The amount of the nickel compound to be used is usually 0.4 to 5 moles relative to 1 mole of the monomer in the monomer composition. Herein, the monomer in the monomer composition means a monomer which is involved in the polymerization reaction and which is contained in the monomer composition such as the dihalobenzene compound, the compound (5) and the compound (6).

The polymerization reaction is preferably conducted in the presence of the nickel compound and a nitrogen-containing bidentate ligand. Examples of the nitrogen-containing bidentate ligand include 2,2'-bipyridine, 1,10-phenanthroline, methylenebisoxazoline and N,N'-tetramethylethylenediamine, and 2,2'-bipyridine is preferable. When the nitrogen-containing bidentate ligand is used, the amount thereof is usually 0.2 to 2 moles, and preferably 1 to 1.5 moles relative to 1 mole of the nickel compound.

When the divalent nickel compound is used as the nickel compound, zinc is usually used together. As zinc, powdery one is usually used. When zinc is used, the amount thereof is usually 1 mole or more relative to 1 mole of the monomers in the monomer composition. The upper limit is not particularly limited, and when it is too much, it may be trouble in the aftertreatment after the polymerization reaction and it may also result in economical disadvantage, and therefore, it is practically 10 moles or less, and preferably 5 moles or less.

When the zerovalent nickel compound is used as the nickel compound and the amount of the zerovalent nickel compound is less than 1 mole relative to 1 mole of the monomers in the monomer composition, the polymerization reaction is usually conducted in the presence of zinc. The powdery zinc is usually used. When zinc is used, the amount thereof is usually 1 mole or more relative to 1 mole of the monomer in the monomer composition, and the upper limit is not particularly limited. When it is too much, it may be trouble in the aftertreatment after the polymerization reaction and it may also result in economical disadvantage, and therefore, it is practically 10 moles or less, and preferably 5 moles or less.

The polymerization reaction is usually carried out in the presence of a solvent. The solvent may be one in which the monomer composition and the polyarylene produced can be dissolved. Specific examples of the solvent include an aromatic hydrocarbon solvent such as toluene and xylene; an ether solvent such as tetrahydrofuran and 1,4-dioxane; an aprotic polar solvent such as dimethylsulfoxide, N-methyl-pyrrolidone, N,N-dimethylformamide, N,N-dimethylacetamide and hexamethylphosphoric triamide; and a halogenated hydrocarbon solvent such as dichloromethane and dichloroethane. These solvents are used alone, and two or more thereof are mixed each other to be used. Among them, the ether solvent and the aprotic polar solvent are preferable and tetrahydrofuran, diemthylsulfoxide, N-methyl-2-pyrrolidone and N,N-dimethylacetoamide are more preferable. When the amount of the solvent is too large, a polyarylene having small molecular weight tends to be obtained, and when the amount thereof is too small, the property of the reaction mixture tends to be bad, and therefore, the amount thereof is usually 1 to 200 parts by weight and preferably 5 to 100 parts by weight relative to 1 parts by weight of the monomers in the monomer composition.

The polymerization reaction is usually conducted in an atmosphere of an inert gas such as nitrogen gas.

The polymerization temperature is usually 0 to 250°C, and preferably 30 to 100°C. The polymerization time is usually 0.5 to 48 hours.

After the completion of polymerization reaction, for example, the polyarylene can be isolated by mixing a solvent in which the polyarylene produced is poorly soluble with the reaction mixture to precipitate the polyarylene and separating the polyarylene precipitated from the reaction mixture by filtration. A solvent in which the polyarylene produced is insoluble or poorly soluble may be mixed with the reaction mixture, and then an aqueous acid solution such as hydrochloric acid may be added thereto followed by separating the polyarylene precipitated by filtration. The molecular weight and the structure of the polyarylene obtained can be analyzed by a conventional means such as gel permeation chromatography and NMR. Examples of the solvent in which the polyarylene produced is insoluble or poorly soluble include water, methanol, ethanol and acetonitrile, and water and methanol are preferable.

Next, a method for converting the polyarylene comprising the repeating unit (2) to a polyarylene comprising a repeating unit represented by the formula (7): 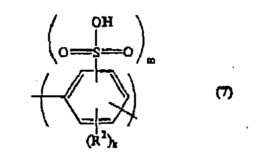 wherein R¹, m and k are the same meanings as above (hereinafter, simply referred to as the repeating unit (7)), will be illustrated.

Examples of the method for converting the polyarylene comprising the repeating unit (2) to the polyarylene comprising the repeating unit (7) include a method comprising hydrolyzing the polyarylene comprising the repeating unit (2) in the presence of an acid or an alkali, and a method comprising reacting the polyarylene comprising the repeating unit (2) with an alkali metal halide or a quaternary ammonium halide followed by conducting an acid treatment.

The polyarylene consisting of the repeating unit (2) can be converted to the polyarylene consisting of the repeating unit (7) by thus method, and the polyarylene comprising the repeating unit (2) and the segment (3) can be converted to a polyarylene comprising the repeating unit (7) and the segment (3). The polyarylene comprising the repeating unit (2) and the repeating unit (4) can be converted to a polyarylene comprising the repeating unit (7) and the repeating unit (4).

The method comprising hydrolyzing the polyarylene comprising the repeating unit (2) in the presence of an acid or an alkali will be illustrated below.

The hydrolysis reaction of the polyarylene comprising the repeating unit (2) is usually conducted by mixing the polyarylene comprising the repeating unit (2) with an aqueous acid or alkali solution. Examples of the aqueous acid solution include an aqueous solution of an inorganic acid such as hydrochloric acid, sulfuric acid and nitric acid, and examples of the aqueous alkali solution include an aqueous solution of an alkali metal hydroxide such as sodium hydroxide and potassium hydroxide. The aqueous acid solution is preferably used and hydrochloric acid is more preferably used. The amount of the acid or alkali may be usually 1 mole or more relative to 1 mole of the group represented by -SO₂A in the polyarylene comprising the repeating unit (2), and the upper limit is not particularly limited.

The hydrolysis reaction may be conducted in the presence of a solvent, and examples of the solvent include a hydrophilic alcohol solvent such as methanol and ethanol. The amount of the solvent to be used is not particularly limited.

The hydrolysis temperature is usually 0 to 250°c and preferably 40 to 120°C. The hydrolysis time is usually 1 to 48 hours.

The progress of the reaction can be confirmed by, for example, NMR or IR.

When the polyarylene comprising the repeating unit (2) is hydrolyzing in the presence of the acid, the polyarylene comprising the repeating unit (7) is usually precipitated in the reaction mixture after completion of the hydrolysis reaction, and the polyarylene comprising the repeating unit (7) can be isolated by filtrating the reaction mixture. When the polyarylene comprising the repeating unit (2) is hydrolyzing in the presence of the alkali, the polyarylene comprising the repeating unit (7) can be isolated by mixing the reaction mixture with the acid to acidify the reaction mixture and to precipitate the polyarylene comprising the repeating unit (7) in the reaction mixture followed by filtrating the reaction mixture.

The polyarylene comprising the repeating unit (7) and the segment (3) is obtained by conducting the similar method to the above against the polyarylene comprising the repeating unit (2) and the segment (3). The polyarylene comprising the repeating unit (7) and the repeating unit (4) can be obtained by conducting the similar method to the above against the polyarylene comprising the repeating unit (2) and the segment (4).

Next, the method comprising reacting the polyarylene comprising the repeating unit (2) with the alkali metal halide or the quaternary ammonium halide followed by conducting an acid treatment will be illustrated.

Examples of the alkali metal halide include lithium bromide and sodium iodide, and examples of the quaternary ammonium halide include tetramethylammonium chloride and tetrabutylammonium bromide, and lithium bromide and tetrabutylammonium bromide are preferable.

The amount of the alkali metal halide or the quaternary ammonium halide to be used is usually 1 mole or more relative to 1 mole of the group represented by -SO₂A in the polyarylene comprising the repeating unit (2), and the upper limit is not particularly limited.

The reaction of the polyarylene comprising the repeating unit (2) and the alkali metal halide or the quaternary ammonium halide is usually conducted by mixing the polyarylene comprising the repeating unit (2) with the alkali metal halide or the quaternary ammonium halide in the presence of a solvent. The solvent may be one that can be dissolve the polyarylene comprising the repeating unit (2) and examples of the solvent include the same as those used in the above-mentioned polymerization reaction. When the amount of the solvent to be used is small, the properties of the reaction mixture may tend to be bad, and when it is too much, the filterability of the polyarylene comprising the repeating unit (7) obtained may tend to be bad, and therefore, it is usually 1 to 200 parts by weight relative to 1 part of the polyarylene comprising the repeating unit (2), and preferably 5 to 50 parts by weight.

The reaction temperature is usually 0 to 250°C, and preferably 100 to 160°C. The reaction time is usually 1 to 48 hours.

The progress of the reaction can be confirmed by NMR or IR.

After completion of the reaction, the polyarylene comprising the repeating unit (7) can be isolated by conducting the acid treatment of the reaction mixture followed by filtration.

The acid treatment is usually carried out by mixing the reaction mixture with an acid. Examples of the acid include hydrochloric acid and sulfuric acid. The amount of the acid may be enough amount to acidify the reaction mixture.

The polyarylene consisting of the repeating unit (7) is obtained by conducting the similar method to the above against the polyarylene consisting of the repeating unit (2). The polyarylene comprising the repeating unit (7) and the segment (3) is obtained by conducting the similar method to the above against the polyarylene comprising the repeating unit (2) and the segment (3). The polyarylene comprising the repeating unit (7) and the repeating unit (4) can be obtained by conducting the similar method to the above against the polyarylene comprising the repeating unit (2) and the segment (4).

An ion-exchange capacity of the polyarylene comprising the repeating unit (7) or the polyarylene consisting of the repeating unit (7), which is measured by titration method, is usually 0.5 to 8.5 meq/g.

Finally, a method for producing the dihalobenzene compound (1) will be illustrated.

The dihalobenzene compound (1) can be produced by reacting a compound represented by the formula (8): 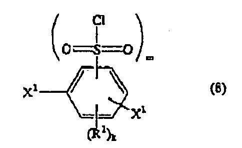 wherein R¹, X¹, m and k are the same as the above (hereinafter, simply referred to as the compound (8)), with a compound represented by the formula (9):

A-H (9)

wherein A is the same as the above (hereinafter, simply referred to as the compound (9)) in the presence of a tertiary amine compound or a pyridine compound.

Examples of the compound (8) include 2,5-dichlorobenzenesulfonyl chloride, 3,5-dichlorobenzenesulfonyl chloride, 2,5-dibromobenzenesulfonyl chloride and 3,5-dibromobenzenesulfonyl chloride. As the compound (8), a commercially available one is usually used.

Examples of the compound (9) include isopropanol, isobutanol, 2,2-dimethylpropanol, cyclohexanol, n-octanol, n-pentadecanol, and n-icosanol. As the compound (9), a commercially available one is usually used.

The amount of the compound (9) is usually 0.2 mole or more relative to 1 mole of the group represented by -SO₂Cl in the compound (8) and there is no specific upper limit. When the compound (9) is a liquid at the reaction temperature, large excess thereof may be used also to serve as the reaction solvent. The practical amount of the compound (9) is 0.5 to 2 moles relative to 1 mole of the group represented by -SO₂Cl in the compound (8).

Examples of the tertiary amine compound include trimethylamine, triethylamine, tri(n-propyl)amine, tri(n-butyl)amine, diisopropylethylamine, tri(n-octyl)amine, tri(n-decyl)amine, triphenylamine, N,N-dimethylaniline, N,N,N' ,N' -tetramethylethylenediamine and N-methylpyrrolidine. A commercially available tertiary amine compound is usually used. The amount of the tertiary amine compound is usually 1 mole or more relative to 1 mole of the group represented by -SO₂Cl in the compound (8) and there is no specific upper limit. When the tertiary amine compound is a liquid at the reaction temperature, large excess thereof may be used also to serve as the reaction solvent. The practical amount of the tertiary amine compound is 1 to 30 moles, preferably 1 to 20 moles and more preferably 1 to 10 moles relative to 1 mole of the group represented by -SO₂Cl in the compound (8).

Examples of the pyridine compound include pyridine and 4-dimethylaminopyridine. A commercially available pyridine compound is usually used. The amount of the pyridine compound is usually 1 mole or more relative to 1 mole of the group represented by -SO₂Cl in the compound (8) and there is no specific upper limit. When the pyridine compound is a liquid at the reaction temperature, large excess thereof may be used also to serve as the reaction solvent. The practical amount of the pyridine compound is 1 to 30 moles, preferably 1 to 20 moles and more preferably 1 to 10 moles relative to 1 mole of the group represented by -SO₂Cl in the compound (8).

The reaction of the compound (8) and the compound (9) is usually conducted by mixing the compound (8), the compound (9) and the tertiary amine compound or the pyridine compound in the presence of the solvent. The mixing order is not particulary limited.

Examples of the solvent include an aromatic hydrocarbon solvent such as toluene and xylene; an ether solvent such as diethyl ether, tetrahydrofuran and 1,4-dioxane; an aprotic polar solvent such as dimethylsulfoxide, N-methyl-2-pyrrolidone, N,N-dimethylformamide, N,N-dimethylacetamide and hexamethylphosphoric triamide; a halogenated hydrocarbon solvent such as dichloromethane, chloroform, dichloroethane, chlorobenzene and dichlorobenzene. As described above, when the compound (9), the tertiary amine compound or the pyridine compound is a liquid at the reaction temperature, they may be used as the reaction solvent. The solvent may be used alone and two or more kinds thereof may be mixed and used. The amount of the solvent is not particularly limited.

The temperature of the reaction of the compound (8) with the compound (9) is usually -30 to 150°C, and preferably -10 to 70°C. The reaction time is usually 0.5 to 24 hours.

After completion of the reaction, for example, an organic layer containing the dihalobenzene compound (1) can be obtained by adding water or an aqueous acid solution and if necessary, a water-insoluble organic solvent to the reaction mixture followed by extraction. The dihalobenzene compound (1) can be isolated by concentrating the organic layer obtained, if necessary, after washing with water or an aqueous alkali solution. The dihalobenzene compound (1) isolated may be further purified by a conventional means such as silica gel chromatography and recrystallization.

Examples of the water-insoluble organic solvent include an aromatic hydrocarbon solvent such as toluene and xylene; an aliphatic hydrocarbon solvent such as hexane and heptane; a halogenated hydrocarbon solvent such as dichloromethane, dichloroethane and chloroform; and an ester solvent such as ethyl acetate. The amount thereof is not particularly limited.

The dihalobenzene compound (1) can also be produced by reacting the compound (8) with a compound represented by the formula (10):

A-M (10)

wherein A is the same meaning as above and M represents an alkali metal atom (hereinafter, simply referred to as the compound (10)).

Examples of the alkali metal atom include lithium, sodium, potassium and cesium, and lithium and sodium are preferable.

Examples of the compound (10) include lithium isopropoxide, lithium isobutoxide, lithium 2,2-dimethylpropoxide, lithium cyclohexyloxide, sodium isobutoxide and potassium isobutoxide. As the compound (10), a commercially available one may be used and one produced according to known methods may be used.

The amount of the compound (10) is usually 0.2 to 2 moles relative to 1 mole of the group represented by -SO₂Cl in the compound (8).

The reaction of the compound (8) with the compound (10) is usually conducted by mixing the compound (8) with the compound (10) in the presence of a solvent. The mixing order is not particularly limited.

Examples of the solvent include an aromatic hydrocarbon solvent such as toluene and xylene; an ether solvent such as diethyl ether, tetrahydrofuran and 1,4-dloxane; an aprotic polar solvent such as dimethylsulfoxide, N-methyl-2-pyrrolidone, N,N-dimethylformamide, N,N-dimethylacetamide and hexamethylphosphoric triamide; a halogenated hydrocarbon solvent such as dichloromethane, chloroform, dichloroethane, chlorobenzene and dichlorobenzene. The solvent may be used alone and two or more kinds thereof may be mixed and used. The amount of the solvent is not particularly limited.

The temperature of the reaction of the compound (8) with the compound (10) is usually -30 to 150°C, and preferably -10 to 70°C. The reaction time is usually 0.5 to 24 hours.

After completion of the reaction, an organic layer containing the dihalobenzene compound (1) can be obtained by adding water and if necessary, a water-insoluble organic solvent to the reaction mixture followed by extraction. The dihalobenzene compound (1) can be isolated by concentrating the organic layer obtained, if necessary, after washing with water or an aqueous alkali solution. The dihalobenzene compound (1) isolated may be further purified by a conventional means such as silica gel chromatography and recrystallization.

Examples of the water-insoluble organic solvent include the same as described above.

### Examples

The present invention will be further illustrated by Examples in detail below, but the present invention is not limited by these Examples. The polyarylenes obtained were analyzed with gel permeation chromatography (the analytical conditions were as followings), and the weight-average molecular weight (Mw) and number-average molecular weight (Mn) were calculated based on the results thereof.

### <Analytical conditions>

### Measuring apparatus: CTO-10A (manufactured by Shimadzu Corporation)

Column: TSK-GEL (manufactured by Tosoh Coporation)
Column temperature: 40°C
Eluent: N,N-dimethylacetamide containing lithium bromide (cnoncentration of lithium bromide: 10 mmol/dm³)
Flow rate: 0.5 mL/min.
Detection wavelength: 300 nm

### Example 1

44.9 G of 2,2-dimethylpropanol was dissolved in 145 g of pyridine. 100 G of 2,5-dichlorobenzenesulfonyl chloride was added thereto at 0°C to stir at room temperature for 1 hour to effect reaction. To the reaction mixture, 740 mL of ethyl acetate and 740mL of 2 mol% hydrochloric acid were added to stir for 30 minutes followed by leaving, and then an organic layer was separated. The organic layer separated was washed with 740 mL of water, 740 mL of 10% by weight aqueous potassium carbonate solution and 740 ml of aqueous saturated sodium chloride solution in this order, and then the solvent was distilled away under reduced pressure condition. The residue was purified with silica gel chromatography (solvent: chloroform). The solvent was distilled away form the eluate obtained under reduced pressure condition. The residue was dissolved in 970 mL of hexane at 65°C followed by cooling to room temperature. The solids precipitated were separated by filtration. The solids separated were dried to obtain 99.4 g of white solids of 2,2-dimethylpropyl 2,5-dichlorobebzenesulfonate. Yield: 82.1%.
¹H-NMR (CDCl₃, δ (ppm)): 0.97 (s, 9H), 3.78 (s, 2H), 7.52-7.53 (c. 2H), 8.07 (d, 1H)
mass spectrum (m/z): 297 (M⁺)

### Example 2

5.1 G of cyclohexanol was dissolved in 14.5 g of pyridine. 10 G of 2,5-dichlorobenzenesulfonyl chloride was added thereto at 0°C to stir at room temperature for 1 hour to effect reaction. To the reaction mixture, 74 mL of ethyl acetate and 74mL of 2 mol% hydrochloric acid were added to stir for 30 minutes followed by leaving, and then an organic layer was separated. The organic layer separated was washed with 74 mL of water, 74 mL of 10% by weight aqueous potassium carbonate solution and 74 ml of aqueous saturated sodium chloride solution in this order, and then the solvent was distilled away under reduced pressure condition. The residue was dissolved in 120 mL of hexane at 65°C followed by cooling to room temperature. The solids precipitated were separated by filtration. The solids separated were dried to obtain 6.0 g of white solids of cyclohexyl 2,5-dichlorobebzenesulfonate. Yield: 47.7%.
¹H-NMR (CDCl₃, δ (ppm)): 1.21-1.86 (c, 10H), 4.68 (dt, 1H), 7.48 (d, 2H), 8.10 (s, 1H)

### Example 3 (reference)

5.7 G of n-dodecyamine and 7.3 g of pyridine were dissolved in 75 mL of chloroform. 5 G of 2,5-dichlorobenzenesulfonyl chloride was added thereto at 0°C to stir at room temperature for 1 hour to effect reaction. To the reaction mixture, 22 mL of chloroform and 40 mL of 2 mol% hydrochloric acid were added to stir for 30 minutes followed by leaving, and then an organic layer was separated. The organic layer separated was washed with 40 mL of water, 40 mL of 10% by weight aqueous potassium carbonate solution and 40 ml of aqueous saturated sodium chloride solution in this order, and then the solvent was distilled away under reduced pressure condition. The residue was purified with silica gel chromatography (solvent: chloroform) . The solvent was distilled away form the eluate obtained under reduced pressure condition. The residue was dissolved in 70 mL of hexane at 65°C followed by cooling to room temperature. The solids precipitated were separated by filtration. The solids separated were dried to obtain 5.3 g of white solids of N,N-n-dodecyl-2,5-dichlorobebzenesulfonamide. Yield: 66.0%.
¹H-NMR (CDCl₃, δ (ppm)): 0.88 (t, 3H), 1.21-1.30 (c, 16H), 1.41-1.49 (c, 2H), 2.94 (dt, 2H), 4.94 (t, 1H), 7.46-7.49 (c, 2H), 8.08 (d, 1H)
mass spectrum (m/z): 394 (M⁺)

### Example 4

0.9 G of 2,2-dimethylpropanol was dissolved in 5.8 g of pyridine. 2 G of 3,5-dichlorobenzenesulfonyl chloride was added thereto at 0°C to stir at room temperature for 1 hour to effect reaction. To the reaction mixture, 30 mL of ethyl acetate and 30mL of 2 mol% hydrochloric acid were added to stir for 30 minutes followed by leaving, and then an organic layer was separated. The organic layer separated was washed with 30 mL of water, 30 mL of 10% by weight aqueous potassium carbonate solution and 30 ml of aqueous saturated sodium chloride solution in this order, and then the solvent was distilled away under reduced pressure condition. The residue was purified with silica gel chromatography (solvent: chloroform). The solvent was distilled away form the eluate obtained under reduced pressure condition to obtain 2.22 g of white solids of 2,2-dimethylpropyl 3,5-dichlorobebzenesulfonate. Yield: 90.9%.
¹H-NMR (CDCl₃), δ (ppm)): 0.91 (s, 9H), 3.72 (s, 2H), 7.63 (t, 1H), 7.78 (d, 2H)
mass spectrum (m/z): 297 (M⁺)

### Example 5

13ML of a hexane solution of n-butyl lithium (1.57M) was added dropwise at 0°C to a solution obtained by dissolving 1.8g of isobutanol in 20 mL of tetrahydrofuran to stir at room temperature for 1 hour to prepare a solution containing lithium butoxide. To the solution obtained by dissloving 4 g of 2,5-dichlorobenzenesulfonyl chloride in 30 mL of tetrahydrofuran, the solution containing lithium butoxide prepared was added dropwise followed by stirring at room temperature for 1 hour to effect reaction. The reaction mixture was concentrated and 40 mL of ethyl acetate and 40mL of water were added to the residue. The mixture was stirred for 30 minutes followed by leaving, and then an organic layer was separated. The organic layer separated was washed with 40 mL of aqueous saturated sodium chloride solution, and then a part of the solvent was distilled away under reduced pressure condition to obtain 7.8 g of concentrate. At 20°C, 7.8 g of hexane was added to the residue and the solids precipitated were separated by filtration. The solids separated were dried to obtain 1.71 g of white solids of isobutyl 2,5-dichlorobebzenesulfonate. Yield: 37.2%.
¹H-NMR (CDCl₃, δ (ppm)): 0.96 (d, 6H), 1.94-2.12 (c, 2H), 3.91 (d, 2H), 7.49-7.56 (c, 2H), 8.04 (s, 1H)
mass spectrum (m/z): 282 (M⁺)

### Example 6

22.4 G of 2,2-dimethylpropanol was dissolved in 72.5 g of pyridine. 50 G of 2,5-dichlorobenzenesulfonyl chloride was dded thereto at 0°C to stir at room temperature for 1 hour to effect reaction. To the reaction mixture, 100 mL of toluene and 250mL of 2 mol% hydrochloric acid were added to stir for 30 minutes followed by leaving, and then an organic layer was separated. The organic layer separated was washed with 150 mL of water, 150 mL of 10% by weight aqueous potassium carbonate solution and 150 mL of water in this order, and then a part of the solvent was distilled away under reduced pressure condition to obtain 105 g of a concentrate. The concentrate was cooled at 0°C, and the solids precipitated were separated by filtration. The solids separated were dried to obtain 49.3 g of white solids of 2,2-dimethylpropyl 2,5-dichlorobebzenesulfonate. Yield: 81.4%.

### Example 7

1.62 G of anhydrous nickel chloride was mixed with 15 mL of dimethylsulfoxide to adjust to an inner temperature of 0°C. To this, 2.15 g of 2,2'-bipyridine was added followed by stirring at the same temperature for 10 minutes to prepare a nickel-containing solution.

To the solution obtained by dissolving 1.49 g of 2,2-dimethylpropyl 2,5-dichlorobenzenesulfonyl chloride and 0.5 g of SUMIKA EXCEL PES 5200P represented by the following formula: 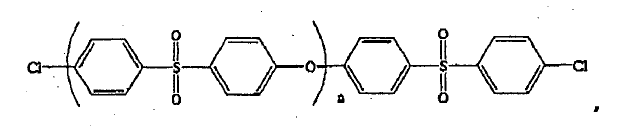 manufactured by Sumitomo Chemical Company, Limited, and Mw=94,000 and Mn=40,000 which were measured by the above analytical conditions, in 5 mL of dimethylsulfoxide, 1.23 g of powdery zinc was added and the mixture was adjusted at 70°C. The above-mentioned nickel-containing solution was poured therein and the polymerization reaction was conducted at 70°C for 4 hours. The reaction mixture was added into 60 mL of methanol and then, 60 mL of 6 mol/L of hydrochloric acid was added thereto to stir for 1 hour. The solids precipitated were separated by filtration and dried to obtain 1.62 g of grayish white polyarylene comprising the repeating unit represented by the following 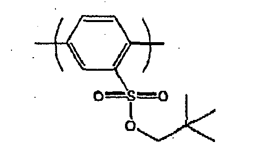 and the segment represented by the following 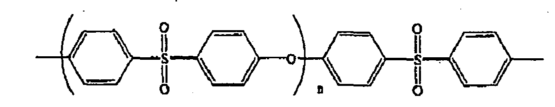 Yield: 99%.
Mw=191,000, Mn=69,000
¹H-NMR (CDCl₃, δ (ppm)): 0.80-1.05 (br), 3.80-3.89 (br), 7.25 (d), 7.97 (d), 7.00-8.50 (c)

### Example 8

3.89 G of anhydrous nickel chloride was mixed with 36 mL of dimethylsulfoxide to adjust to an inner temperature of 0°C. To this, 5.15 g of 2,2'-bipyridine was added followed by stirring at the same temperature for 10 minutes to prepare a nickel-containing solution.

To the solution obtained by dissolving 3.57 g of 2,2-dimethylpropyl 2,5-dichlorobenzenesulfonyl chloride in 12 mL of dimethylsulfoxide, 2.94 g of powdery zinc was added and the mixture was adjusted at 70°C. The above-mentioned nickel-containing solution was poured therein and the polymerization reaction was conducted at 70°C for 4 hours. The reaction mixture was added into 120 mL of methanol and then, 120 mL of 6 mol/L of hydrochloric acid was added thereto to stir for 1 hour. The solids precipitated were separated by filtration and dried to obtain 2-7 g of grayish white polyarylene consisting of the repeating unit represented by the following 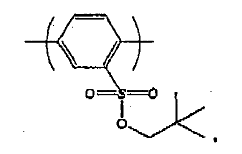 Yield: 99%.
Mw=201,000, Mn=59,000
¹H-NMR ((CD₃)₂SO, δ (ppm)): 0.80-1.05 (br), 3.80-3.89 (br), 7.00-8.50 (c)

### Example 9

0.16 G of anhydrous nickel chloride was mixed with 1.5 mL of dimethylsulfoxide to adjust to an inner temperature of 0°C. To this, 0.22 g of 2,2'-bipyridine was added followed by stirring at the same temperature for 10 minutes to prepare a nickel-containing solution.

To the solution obtained by dissolving 0.15 g of 2,2-dimethylpropyl 3,5-dichlorobenzenesulfonyl chloride in 0.5 mL of dimethylsulfoxide, 0.12 g of powdery zinc was added and the mixture was adjusted at 70°C. The above-mentioned nickel-containing solution was poured therein and the polymerization reaction was conducted at 70°C for 4 hours to obtain the reaction mixture containing the polyarylene consisting of the repeating unit represented by the following 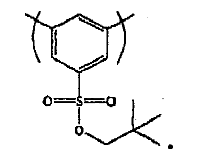 Mw of the polyarylene was 199,000 and Mn thereof was 93,000.

### Example 10 (reference)

According to a similar manner as that of Example 9, the reaction mixture containing the polyarylene consisting of the repeating unit represented by the following 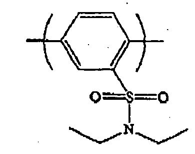 was obtained except that 0.14 g of N,N-diethyl-2,5-dichlorobenzenesulfonamide was used in place of 0.15 g of 2,2-dimethylpropyl 3,5-dichlorobenzenesulfonyl chloride. Mw of the polyarylene was 7,200 and Mn thereof was 2,700.

### Example 11

According to a similar manner as that of Example 9, the reaction mixture containing the polyarylene consisting of the repeating unit represented by the following 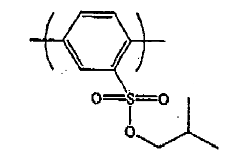 was obtained except that 0.14 g of isobutyl 2,5-dichlorobenzenesulfonate was used in place of 0.15 g of 2,2-dimethylpropyl 3,5-dichlorobenzenesulfonyl chloride. Mw of the polyarylene was 7,400 and Mn thereof was 4,500.

### Example 12

To the reaction glass vessel equipped with a cooling apparatus, 168 mg of bis(octadiene)nickel(0), 105 mg of 2,2'-bipyridine, 100 mg of powdery zinc and 4 mL of N-methyl-2-pyrrolidone were added in an atmosphere of nitrogen to stir at 70°C for 30 minutes. To this, the solution obtained by dissolving 217 mg of isobutyl 2,5-dichlorobenzenesulfonyl chloride in 1 mL of N-methyl-2-pyrrolidone was added and the polymerization reaction was conducted at 70°C for 4 hours to obtain the reaction mixture containing the polyarylene consisting of the repeating unit represented by the following 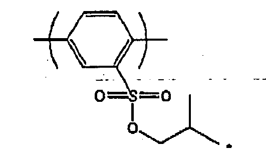 Mw of the polyarylene was 34,000 and Mn thereof was 19,000.

### Example 13

The mixture of 60 mL of tetrahydrofuran, 0.89 g of 2,2-dimethylpropyl 2,5-dichlorobenzenesulfonate and 1. 29 g of 2,2'-bipyridine was adjusted at 70°C. To this, 2.06 g of bis(cyclooctadiene)nickel(0) was added and the polymerization reaction was conducted for 4 hours to obtain the reaction mixture containing the polyarylene consisting of the repeating unit represented by the following 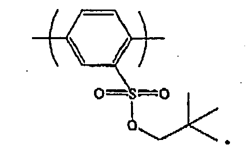 Mw of the polyarylene was 433,000 and Mn thereof was 251,000.

### Example 14

The solution obtained by dissolving 2.28 g of 2,2-dimethylpropyl 2,5-dichlorobenzenesulfonate in 25 mL of N-methyl-2-pyrrolidone was adjusted at 70°C. Into this, the solution obtained by dissolving 4.21 g of bis(cycloootadiene)nickel(0) and 2,2'-bipyridine in 25 mL of N-methyl-2-pyrrolidone (inner temperature was 70°C) was poured and the polymerization reaction was conducted at 70°C for 8 hours to obtain the reaction mixture containing the polyarylene consisting of the repeating unit represented by the following 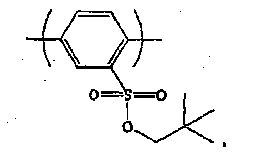 Mw of the polyarylene was 91,000 and Mn thereof was 50,000.

### Example 15

To the mixed solution of 0.23 g of anhydrous nickel chloride and 3.6 mL of dimethylsulfoxide, which was adjusted at an inner temperature of 70°C, 0.31 g of 2,2'-bipyridine was added to stir at the same temperature for 10 minute to prepare the nickel-containing solution. To the solution obtained by dissolving 0.36 g of 2,2-dimethylpropyl 2,5-dichlorobenzenesulfonate in 1.2 mL of dimethylsulfoxide, 0.29 g of powdery zinc was added followed by adjusting at 70°C. Into this, the above-mentioned, nickel-containing solution was poured, and the polymerization reaction was conducted at 70°C for 4 hours to obtain the reaction mixture containing the polyarylene consisting of the repeating unit represented by the following 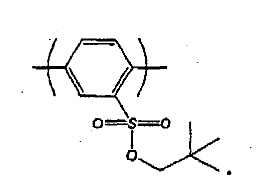 Mw of the polyarylene was 56,000 and Mn thereof was 27,000.

### Example 16

To the reaction glass vessel equipped with a cooling apparatus, 1.68 g of bis(octadiene)nickel(0), 0.96 g of 2,2'-bipyridine and 20 mL of N-methyl-2-pyrrolidone were added in an atmosphere of nitrogen to stir at 70°C for 30 minutes to obtain the nickel-containing solution. To the reaction glass vessel equipped with a cooling apparatus, 2.28 g of 2,2-dimethylpropyl 2,5-dichlorobenzenesulfonate, 125 g of powdery zinc and 30 mL of N-methyl-2-pyrrolidone were added in an atmosphere of nitrogen followed by adjusting at an inner temperature of 70°C. The above-mentioned nickel-containing solution was poured into this, and the polymerization reaction was conducted at 70°C for 8 hours to obtain the reaction mixture containing the polyarylene consisting of the repeating unit represented by the following 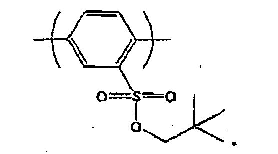 Mw of the polyarylene was 77,000 and Mn thereof was 36,000.

### Example 17

To the reaction glass vessel equipped with a cooling apparatus, 84 mg of nickel bromide, 66 mg of 2,-2'-bipyridine, 100 mg of powdery zinc and 4 mL of N,N-dimethylacetamide were added in an atmosphere of nitrogen at room temperature to prepare the nickel-containing solution. To this, the solution obtained by dissolving 227 mg of 2,2-dimethylpropyl 2,5-dichlorobenzenesulfonate in 1 mL of N,N-dimethylacetamide was added, and the polymerization reaction was conducted at 70°C for 4 hours to obtain the reaction mixture containing the polya-rylens consisting of the repeating unit represented by the following 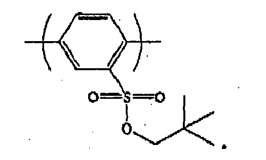 Mw of the polyarylene was 67,000 and Mn thereof was 23,000.

### Example 18

To the reaction glass vessel equipped with a cooling apparatus, 5.05 g of bis(cyclooctadiene)nickel(0), 2.87 g of 2,2'-bipyridine and 40 mL of N-methyl-2-pyrrolidone were added in an atmosphere of nitrogen to stir at 70°C for 30 minutes to prepare the nickel-containing solution. To the reaction glass vessel equipped with a cooling apparatus, 9.09 g of 2,2-dimethylpropyl 2,5-dichlorobenzenesulfonate, 2.4 g of powdery zinc and 40 mL of N-methyl-2-pyrrolidone were added in an atmosphere of nitrogen followed by adjusting at 70°C. The above-mentioned nickel-containing solution was poured into this, and the polymerization reaction was conducted at 70°C. After 1.5 hours from the starting of the polymerization reaction, the solution obtained by dissolving 3.06 g of SUMIKA EXCEL PES 5200P represented by the following formula: 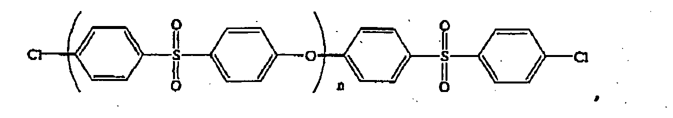 manufactured by Sumitomo Chemical Company, Limited, and Mw=94,000 and Mn=40,000, which were measured by the above analytical conditions, in 40 mL of N-methyl-2-pyrrolidone (inner temperature was 70°C) was added to the reaction mixture and further, the polymerization reaction was conducted at 70°C for 6.5 hours. After completion of the reaction, the reaction mixture was added into 300 mL of methanol and then, 300 mL of 6 mol/L of hydrochloric acid was added thereto to stir for 1 hour. The solids precipitated were separated by filtration and dried to obtain 8.75 g of grayish white polyarylene comprising the repeating unit represented by the following 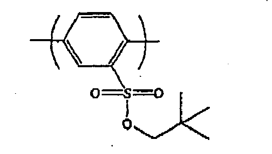 and the segment represented by the following 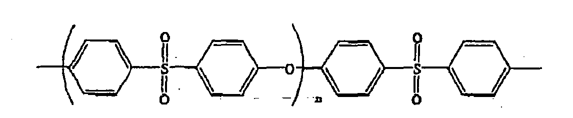 Yield: 87%.
Mw=192,000, Mn=49,000
¹H-NMR (CDCl₃, δ (ppm)): 0.80-1.05 (br), 3.80-3.89 (br), 7.25 (d), 7.97 (d), 7.00-8.50 (c)

### Example 19

To the reaction glass vessel equipped with a cooling apparatus, 168 mg of bis(cyclooctadiene)nickel(0), 105 mg of 2,2'-bipyridine, 100 mg of powdery zinc and 4 mL of N-methyl-2-pyrrolidone were added in an atmosphere of nitrogen to stir at 70°C for 30 minutes to prepare the nickel-containing solution. The solution obtained by dissolving 114 mg of 2,2-dimethylpropyl 2,5 -dichlorobenzenesulfonate a and 56 mg of 1,4-dichlorobenzene in 1 mL of N-methyl-2-pyrrolidone was added thereto. The polymerization reaction was conducted at 70°C for 4 hours to obtain the reaction mixture containing the polyarylene comprising the repeating unit represented by the following 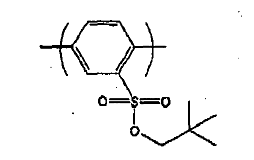 and the repeating unit represented by the following 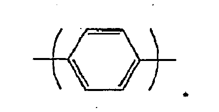 Mw of the polyarylene was 50,000, and Mn thereof was 22,000.

### Example 20

To the reaction glass vessel equipped with a cooling apparatus, 168 mg of bis(cyclooctadiene)nickel(0), 105 mg of 2,2'-bipyridine, 100 mg of powdery zinc and 4 mL of N-methyl-2-pyrrolidone were added in an atmosphere of nitrogen to stir at 70°C for 30 minutes to prepare the nickel-containing solution. The solution obtained by dissolving 114 mg of 2,2-dimethylpropyl 2,5-dichlorobenzenesulfonatea and 131 mg of 2,5-dichloro-4'-phenoxybenzophenone in 1 mL of N-methyl-2-pyrrolidone was added thereto. The polymerization reaction was conducted at 70°C for 4 hours to obtain the reaction mixture containing the polyarylene comprising the repeating unit represented by the following 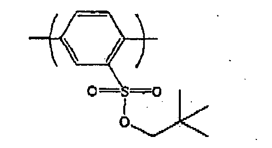 and the repeating unit represented by the following 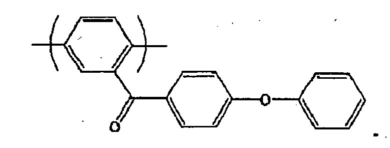 Mw of the polyarylene was 157,000, and Mn thereof was 49,000.

### Example 21

To the mixed solution of 0.16 g of lithium bromide monohydrate and 8 mL of N-methyl-2-pyrrolidone, 0.23 g of the polyarylene obtained in Example 7 was added to effect reaction at 120°C for 24 hours. The reaction mixture was poured into 80 mL of mol/L hydrochloric acid to stir for 1 hour. The solids precipitated were separated by filtration. The solids separated were dried to obtain 0.06 g of the grayish white polyarylene comprising the repeating unit represented by the following 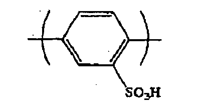 and the segment represented by the following 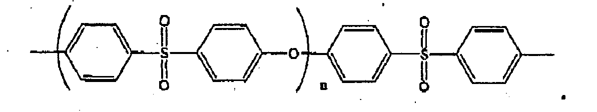 IR spectrum and ¹H-NMR spectrum were measured to confirm that 2,2-dimethylpropoxysulfonyl groups were converted quantitatively to sulfonic acid groups. Mw of the polyarylene obtained was 173,000 and Mn thereof was 75,000. The ion-exchanged capacity was measured by the titration method to find 1.95 meq/g.
¹H-NMR ((CD₃)₂SO₂., δ (ppm)): 7.25 (d), 7.97 (d), 7.00-8.50 (c)

### Example 22

To the mixed solution of 0.16 g of lithium bromide monohydrate and 8 mL of N-methyl-2-pyrrolidone, 0.23 g of the polyarylene obtained in Example 8 was added to effect reaction at 120°C for 24 hours. To the reaction mixture, 10 mL of 6 mol/L hydrochloric acid was added to stir at room temperature for 1 hour. The mixture obtained was poured into 80 mL of acetonitrile and the solids precipitated were separated by filtration. The solids separated were dried to obtain 0.14 g of the grayish white polyarylene consisting of the repeating unit represented by the following 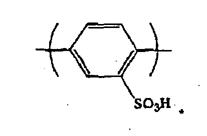 IR spectrum and ¹H-NMR spectrum were measured to confirm that 2,2-dimethylpropoxysulfonyl groups were converted quantitatively to sulfonic acid groups. Mw of the polyarylene obtained was 214,000 and Mn thereof was 105,000.
¹H-NMR ((CD₃)₂SO₂, δ (ppm)): 7.00-8.50 (c)

### Example 23

To the mixed solution of 4.8 g of lithium bromide monohydrate and 90 mL of N-methyl-2-pyrrolidone, 8 g of the polyarylene obtained in Example 18 was added to effect reaction at 120°C for 24 hours. The reaction mixture was poured into 500 mL of 6 mol/L hydrochloric acid to stir for 1 hour. The solids precipitated were separated by filtration. The solids separated were dried to obtain 3.7 g of the grayish white polyarylene comprising the repeating unit represented by the following 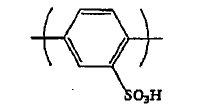 and the segment represented by the following 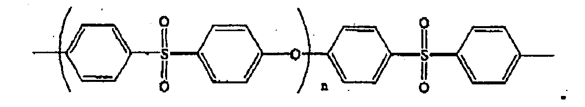 IR spectrum and ¹H-NMR spectrum were measured to confirm that 2,2-dimethylpropoxysulfonyl groups were converted quantitatively to sulfonic acid groups. Mw of the polyarylene obtained was 288,000 and Mn thereof was 83,000. The ion-exchanged capacity was measured by the titration method to find 2.46 meq/g.
¹H-NMR ((CD₃)₂SO₂, δ (ppm)): 7.25 (d), 7.97 (d), 7.00-8.50 (c)

### Industrial Applicability

The dihalobenzene compound of the present invention is useful as a monomer of a polyarylene which can be easily converted to a polyarylene having sulfonic acid groups which is useful as a polyelectrolyte for proton-exchange membrane fuel cell.

## Claims

1. A polyarylene comprising a repeating unit represented by the formula (2): 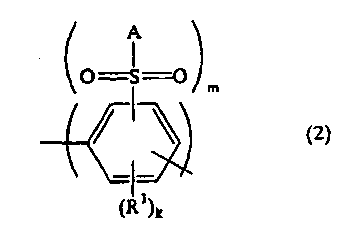 wherein A represents a C3-C20 alkoxy group, which may be substituted with at least one group selected from the group consisting of a fluorine atom, a C1-C20 alkoxy group, a C6-C20 aryl group, a C6-C20 aryloxy group, a C2-C20 acyl group and a cyano group,
R' represents a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the C1-C20 alkyl group, the C1-C20 alkoxy group, the C6-C20 aryl group, the C6-C20 aryloxy group and the C2-C20 acyl group may be substituted with at least one substituent selected from the group consisting of a fluorine atom, a cyano group, a C1-C20 alkoxy group, a C6-C20 aryl group and a C6-C20 aryloxy group, and when multiple R¹s exist, R¹s may be the same groups or different groups, and the neighboring two R¹s may be bonded to form a ring,
X¹ represents a chlorine atom, a bromine atom or an iodine atom, m represents 1 or 2, and k represents 4-m.

2. The polyarylene according to claim 1, wherein m is 1.

3. The polyarylene according to claim 1, wherein R' is a hydrogen atom.

4. A polyarylene consisting of a repeating unit represented by the formula (2) according to claim 1.

5. The polyarylene according to claim 1, wherein the polyarylene comprises a repeating unit represented by the formula (2) according to claim 1 and a segment represented by the formula (3); 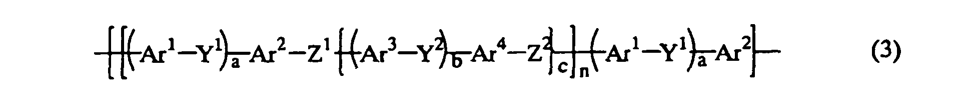 wherein a, b and c represent the same or different, and each represents 0 or 1, and n represents a integer of 5 or more, Ar¹, Ar², Ar³ and Ar⁴ are the same or different, and each represents a divalent aromatic group, and the divalent aromatic group may be substituted with at least one substituent selected from the group consisting of
a C1-C20 alkyl group which may be substituted with at least one selected from the group consisting of a fluorine atom, a cyano group, a C1-C20 alkoxy group, a C6-C20 aryl group and a C6-C20 aryloxy group;
a C1-C20 alkoxy group which may be substituted with at least one selected from the group consisting of a fluorine atom, a cyano group, a C1-C20 alkoxy group, a C6-C20 aryl group and a C6-C20 aryloxy group;
a C6-C20 aryl group which may be substituted with at least one selected from the group consisting of a fluorine atom, a cyano group, a C1-C20 alkoxy group and a C6-C20 aryloxy group;
a C6-C20 aryloxy group which may be substituted with at least one selected from the group consisting of a fluorine atom, a cyano group, a C1-C20 alkoxy group and a C6- C20 aryloxy group; and
a C2-C20 acyl group which may be substituted with at least one selected from the group consisting of a fluorine atom, a cyano group, a C1-C20 alkoxy group, a C6-C20 aryl group and a C6- C20 aryloxy group,
Y¹ and Y² are the same or different, and each represents a single bond, a carbonyl group, a sulfonyl group, 2,2-isopropylidene group, 2,2-hexafluoroisopropylidene group or a fluorene-9,9-diyl group, and
Z¹ and Z² are the same or different, and each represents an oxygen or sulfur atom.

6. The polyarylene according to claim 1, wherein the polyarylene comprises a repeating unit represented by the formula (2) according to claim 1 and a repeating unit represented by the formula (4):
-Ar⁵- (4)
wherein Ar⁵ represents a divalent aromatic group, and the divalent aromatic group may be substituted with at least one substituent selected from the group consisting of
a C1-C20 alkyl group which may be substituted with at least one selected from the group consisting of a fluorine atom. a cyano group, a C1-C20 alkoxy group, a C6-C20 aryl group and a C6-C20 aryloxy group;
a C1-C20 alkoxy group which may be substituted with at least one selected from the group consisting of a fluorine atom, a cyano group, a C1-C20 alkoxy group, a C6-C20 aryl group and a C6-C20 aryloxy group;
a C6-C20 aryl group which may be substituted with at least one selected from the group consisting of a fluorine atom, a cyano group, a C1-C20 alkoxy group and a C6-C20 aryloxy group:
a C6-C20 aryloxy group which may be substituted with at least one selected from the group consisting of a fluorine atom, a cyano group, a C1-C20 alkoxy group and a C6- C20 aryloxy group; and
a C2-C20 acyl group which may be substituted with at least one selected from the group consisting of a fluorine atom, a cyano group, a C1-C20 alkoxy group, a C6-C20 aryl group and a C6- C20 aryloxy group.

7. A method for producing a polyarylene comprising the repeating unit represented by the formula (2) according to claim 1, which comprises polymerizing a monomer composition comprising the dihalobenzene compound represented by the formula (1) 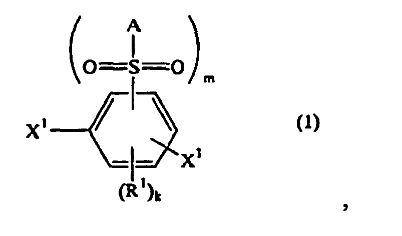 wherein A, R¹, m and k have the same meanings as defined in claim 1, in the presence of a nickel compound.

8. The method for producing a polyarylene according to claim 7, wherein the monomer composition comprises the dihalobenzene compound represented by the formula (1) according to claim 7 and a compound represented by the formula (5): 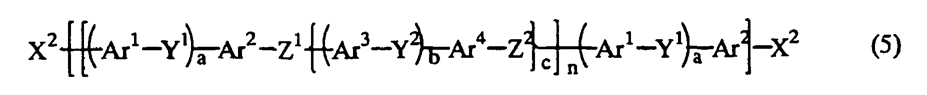 wherein a, b, c, n, Ar¹, Ar², Ar³, Ar⁴, Y¹, Y², Z¹ and Z² represent the same meanings as defined in claim 5 and X² represents a chlorine, bromine or iodine atom.

9. The method for producing a polyarylene according to claim 7, wherein the monomer composition comprises the dihalobenzene compound represented by the formula (1) according to claim 7 and a compound represented by the formula (6):
X³-Ar⁵-X³ (6)
wherein Ar⁵ represents the same meaning as defined in claim 6 and X³ represents a chlorine, bromine or iodine atom.

10. The method for producing a polyarylene in accordance with claim 7, wherein the polyarylene consists of repeating units represented by the formula (2) according to claim 1, which method comprises polymerizing the dihalobenzene compound represented by the formula (1) according to claim 7 only.

11. The method for producing a polyarylene according to any one of claims 7 to 10, wherein the nickel-compound is bis(cyclooctadiene)nickel (0) and the polymerization is conducted in the presence of a nitrogen-containing bidentate ligand.

12. The method for producing a polyarylene according to any one of claims 7 to 10,
wherein the nickel compound is bis(cyclooctadiene)nickel (0) and the polymerization is conducted in the presence of a nitrogen-containing bidentate ligand and zinc.

13. The method for producing a polyarylene according to any one of claims 7 to 10,
wherein the nickel compound is nickel halide and the polymerization is conducted in the presence of a nitrogen-containing bidentate ligand and zinc.

14. A method for producing a polyarylene comprising a repeating unit represented by the formula (7): 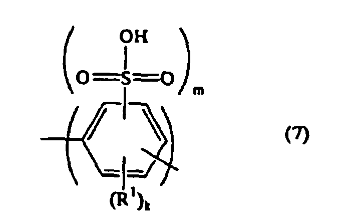 wherein R¹, m and k represent the same meanings as defined in claim 1, which comprises hydrolyzing the polyarylene according to claim 1 in the presence of an acid or an alkali.

15. A method for producing a polyarylene comprising a repeating unit represented by the formula (7) according to claim 14, which comprises reacting the polyarylene according to claim 1 with an alkali metal halide or a quaternary ammonium halide followed by conducting acid treatment.

16. A method for producing a polyarylene comprising the repeating unit represented by the formula (7) according to claim 14 and the segment represented by the formula (3) according to claim 5, which comprises hydrolyzing the polyarylene according to claim 5 in the presence of an acid or an alkali.

17. A method for producing a polyarylene comprising a repeating unit represented by the formula (7) according to claim 14 and the segment represented by the formula (3) according to claim 5, which comprises reacting the polyarylene according to claim 5 with an alkali metal halide or a quaternary ammonium halide followed by conducting acid treatment.

18. A method for producing a polyarylene comprising the repeating unit represented by the formula (7) according to claim 14 and the repeating unit represented by the
formula (4) according to claim 6, which comprises hydrolyzing the polyarylene according to claim 6 in the presence of an acid or an alkali.

19. A method for producing a polyarylene comprising a repeating unit represented by the formula (7) according to claim 14 and the repeating unit represented by the formula (4) according to claim 6, which comprises reacting the polyarylene according to claim 6 with an alkali metal halide or a quaternary ammonium halide followed by conducting acid treatment.

20. A method for producing a polyarylene consisting of the repeating unit represented by the formula (7) according to claim 14, which comprises hydrolyzing the polyarylene according to claim 4 in the presence of an acid or an alkali.

21. A method for producing a polyarylene consisting of the repeating unit represented by the formula (7) according to claim 14, which comprises reacting the polyarylene according to claim 4 with an alkali metal halide or a quaternary ammonium halide followed by conducting acid treatment.

22. The polyarylene according to claim 1, wherein the polyarylene contains the repeating unit represented by the formula (2) according to claim 1 and at least two repeating units represented by the formula (2) according to claim 1 are continued.

## Patentansprüche

1. Ein Polyarylen, umfassend eine Wiederholungseinheit der Formel (2): 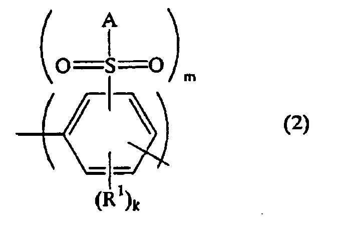 wobei A einen C3-C20 Alkoxyrest darstellt, welcher substituiert sein kann mit wenigstens einem Rest, ausgewählt aus der Gruppe bestehend aus einem Fluoratom, einem C1-C20 Alkoxyrest, einem C6-C20 Arylrest, einem C6-C20 Aryloxyrest, einem C2-C20 Acrylrest und einer Cyanogruppe,
R¹ ein Wasserstoffatom, ein Fluoratom, einen C1-C20 Alkylrest, einen C1-C20 Alkoxyrest, einen C6-C20 Arylrest, einen C6-C20 Aryloxyrest, einen C2-C20 Acylrest oder eine Cyanogruppe darstellt, und der C1-C20 Alkylrest, der C1-C20 Alkoxyrest, der C6-C20 Arylrest, der C6-C20 Aryloxyrest und der C2-C20 Acylrest substituiert sein können mit wenigstens einem Substituenten, ausgewählt aus der Gruppe bestehend aus einem Fluoratom, einer Cyanogruppe, einem C1-C20 Alkoxyrest, einem C6-C20 Arylrest und einem C6-C20 Aryloxyrest, und, falls mehrere R¹ vorliegen, die R¹ dieselben Reste oder unterschiedliche Reste sein können, und zwei benachbarte R¹ miteinander verbunden sein können, um einen Ring zu bilden,
X¹ ein Chloratom, ein Bromatom oder ein Iodatom darstellt, m 1 oder 2 darstellt und k 4-m darstellt.

2. Das Polyarylen gemäß Anspruch 1, wobei m 1 ist.

3. Das Polyarylen gemäß Anspruch 1, wobei R¹ ein Wasserstoffatom ist.

4. Ein Polyarylen, bestehend aus einer Wiederholungseinheit der Formel (2) gemäß Anspruch 1.

5. Das Polyarylen gemäß Anspruch 1, wobei das Polyarylen eine Wiederholungseinheit der Formel (2) gemäß Anspruch 1 und ein Segment der Formel (3) umfasst: 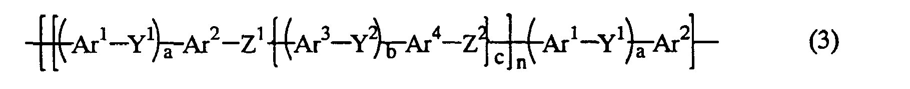 wobei a, b und c gleich oder verschieden sind, und jedes die Bedeutung 0 oder 1 hat, und n eine ganze Zahl von 5 oder mehr darstellt, Ar¹, Ar², Ar³ und Ar⁴ gleich oder verschieden sind, und jedes einen divalenten, aromatischen Rest darstellt, und der divalente, aromatische Rest substituiert sein kann mit wenigstens einem Substituenten, ausgewählt aus der Gruppe bestehend aus
einem C1-C20 Alkylrest, welcher substituiert sein kann mit wenigstens einem Substituenten, ausgewählt aus der Gruppe bestehend aus einem Fluoratom, einer Cyanogruppe, einem C1-C20 Alkoxyrest, einem C6-C20 Arylrest und einem C6-C20 Aryloxyrest;
einem C1-C20 Alkoxyrest, welcher substituiert sein kann mit wenigstens einem Substituenten, ausgewählt aus der Gruppe bestehend aus einem Fluoratom, einer Cyanogruppe, einem C1-C20 Alkoxyrest, einem C6-C20 Arylrest und einem C6-C20 Aryloxyrest;
einem C6-C20 Arylrest, welcher substituiert sein kann mit wenigstens einem Substituenten, ausgewählt aus der Gruppe bestehend aus einem Fluoratom, einer Cyanogruppe, einem C1-C20 Alkoxyrest und einem C6-C20 Aryloxyrest;
einem C6-C20 Aryloxyrest, welcher substituiert sein kann mit wenigstens einem Substituenten, ausgewählt aus der Gruppe bestehend aus einem Fluoratom, einer Cyanogruppe, einem C1-C20 Alkoxyrest und einem C6-C20 Aryloxyrest; und
einem C2-C20 Acylrest, welcher substituiert sein kann mit wenigstens einem Substituenten, ausgewählt aus der Gruppe bestehend aus einem Fluoratom, einer Cyanogruppe, einem C1-C20 Alkoxyrest, einem C6-C20 Arylrest und einem C6-C20 Aryloxyrest;
Y¹ und Y² gleich oder verschieden sind und jedes eine Einfachbindung, einen Carbonylrest, einen Sulfonylrest, 2,2-Isopropylidenrest, 2,2-Hexafluorisopropylidenrest oder einen Fluoren-9,9-diylrest darstellt, und
Z¹ und Z² gleich oder verschieden sind und jedes ein Sauerstoff- oder Schwefelatom darstellt.

6. Das Polyarylen gemäß Anspruch 1, wobei das Polyarylen eine Wiederholungseinheit der Formel (2) gemäß Anspruch 1 und eine Wiederholungseinheit der Formel (4) umfasst:
-Ar⁵- (4)
wobei Ar⁵ einen divalenten, aromatischen Rest darstellt, und der divalente, aromatische Rest substituiert sein kann mit wenigstens einem Substituenten, ausgewählt aus der Gruppe bestehend aus
einem C1-C20 Alkylrest, welcher substituiert sein kann mit wenigstens einem Substituenten, ausgewählt aus der Gruppe bestehend aus einem Fluoratom, einer Cyanogruppe, einem C1-C20 Alkoxyrest, einem C6-C20 Arylrest und einem C6-C20 Aryloxyrest;
einem C1-C20 Alkoxyrest, welcher substituiert sein kann mit wenigstens einem Substituenten, ausgewählt aus der Gruppe bestehend aus einem Fluoratom, einer Cyanogruppe, einem C1-C20 Alkoxyrest, einem C6-C20 Arylrest und einem C6-C20 Aryloxyrest;
einem C6-C20 Arylrest, welcher substituiert sein kann mit wenigstens einem Substituenten, ausgewählt aus der Gruppe bestehend aus einem Fluoratom, einer Cyanogruppe, einem C1-C20 Alkoxyrest und einem C6-C20 Aryloxyrest;
einem C6-C20 Aryloxyrest, welcher substituiert sein kann mit wenigstens einem Substituenten, ausgewählt aus der Gruppe bestehend aus einem Fluoratom, einer Cyanogruppe, einem C1-C20 Alkoxyrest und einem C6-C20 Aryloxyrest; und
einem C2-C20 Acylrest, welcher substituiert sein kann mit wenigstens einem Substituenten, ausgewählt aus der Gruppe bestehend aus einem Fluoratom, einer Cyanogruppe, einem C1-C20 Alkoxyrest, einem C6-C20 Arylrest und einem C6-C20 Aryloxyrest.

7. Ein Verfahren zur Herstellung eines Polyarylens, umfassend die Wiederholungseinheit der Formel (2) gemäß Anspruch 1, welches das Polymerisieren einer Monomerzusammensetzung, umfassend die Dihalobenzolverbindung der Formel (1): 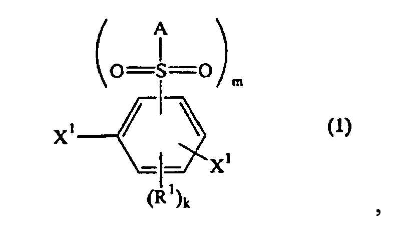 wobei A, R¹, m und k dieselben Bedeutungen wie in Anspruch 1 definiert haben, in Gegenwart einer Nickelverbindung umfasst.

8. Das Verfahren zu Herstellung eines Polyarylens gemäß Anspruch 7, wobei die Monomerzusammensetzung die Dihalobenzolverbindung der Formel (1) gemäß Anspruch 7 und eine Verbindung der Formel (5) umfasst: 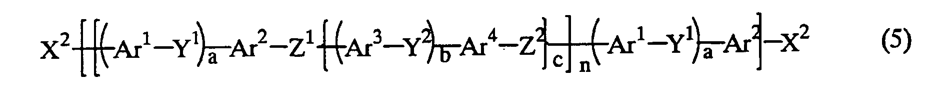 wobei a, b, c, n, Ar¹, Ar², Ar³, Ar⁴, Y¹, Y², Z¹ und Z² dieselben Bedeutungen wie in Anspruch 5 definiert haben und X² ein Chlor-, Brom- oder Iodatom darstellt.

9. Das Verfahren zur Herstellung eines Polyarylens gemäß Anspruch 7, wobei die Monomerzusammensetzung die Dihalobenzolverbindung der Formel (1) gemäß Anspruch 7 und eine Verbindung der Formel (6) umfasst:
X³Ar⁵-X³ (6)
wobei Ar⁵ dieselbe Bedeutung wie in Anspruch 6 definiert hat und X³ ein Chlor-, Brom- oder Iodatom darstellt.

10. Das Verfahren zur Herstellung eines Polyarylens gemäß Anspruch 7, wobei das Polyarylen aus Wiederholungseinheiten der Formel (2) gemäß Anspruch 1 besteht, wobei das Verfahren die Polymerisation lediglich der Dihalobenzolverbindung der Formel (1) gemäß Anspruch 7 umfasst.

11. Das Verfahren zur Herstellung eines Polyarylens gemäß einem der Ansprüche 7 bis 10, wobei die Nickelverbindung Bis(cyclooctadien)nickel(0) ist und die Polymerisation in Gegenwart eines stickstoffhaltigen, zweizähnigen Liganden durchgeführt wird.

12. Das Verfahren zur Herstellung eines Polyarylens gemäß einem der Ansprüche 7 bis 10, wobei die Nickelverbindung Bis(cyclooctadien)nickel(0) ist und die Polymerisation in Gegenwart eines stickstoffhaltigen, zweizähnigen Liganden und von Zink durchgeführt wird.

13. Das Verfahren zur Herstellung eines Polyarylens gemäß einem der Ansprüche 7 bis 10, wobei die Nickelverbindung ein Nickelhalogenid ist und die Polymerisation in Gegenwart eines stickstoffhaltigen, zweizähnigen Liganden und von Zink durchgeführt wird.

14. Ein Verfahren zur Herstellung eines Polyarylens, umfassend eine Wiederholungseinheit der Formel (7): 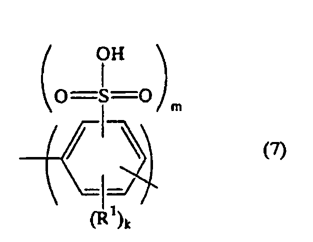 wobei R¹, m und k dieselben Bedeutungen wie in Anspruch 1 definiert haben, welches das Hydrolysieren des Polyarylens gemäß Anspruch 1 in Gegenwart einer Säure oder Base umfasst.

15. Ein Verfahren zur Herstellung eines Polyarylens, umfassend eine Wiederholungseinheit der Formel (7) gemäß Anspruch 14, welches das Umsetzen des Polyarylens gemäß Anspruch 1 mit einem Alkalimetallhalogenid oder einem quartären Ammoniumhalogenid, gefolgt von der Durchführung einer Säurebehandlung, umfasst.

16. Ein Verfahren zu Herstellung eines Polyarylens, umfassend die Wiederholungseinheit der Formel (7) gemäß Anspruch 14 und das Segment der Formel (3) gemäß Anspruch 5, welches das Hydrolysieren des Polyarylens gemäß Anspruch 5 in Gegenwart einer Säure oder Base umfasst.

17. Ein Verfahren zur Herstellung eines Polyarylens, umfassend eine Wiederholungseinheit der Formel (7) gemäß Anspruch 14 und das Segment der Formel 3 gemäß Anspruch 5, welches das Umsetzen des Polyarylens gemäß Anspruch 5 mit einem Alkalimetallhalogenid oder einem quartären Ammoniumhalogenid, gefolgt von der Durchführung einer Säurebehandlung, umfasst.

18. Ein Verfahren zur Herstellung eines Polyarylens, umfassend die Wiederholungseinheit der Formel (7) gemäß Anspruch 14 und die Wiederholungseinheit der Formel (4) gemäß Anspruch 6, welches das Hydrolysieren des Polyarylens gemäß Anspruch 6 in Gegenwart einer Säure oder Base umfasst.

19. Ein Verfahren zur Herstellung eines Polyarylens, umfassend eine Wiederholungseinheit der Formel (7) gemäß Anspruch 14 und die Wiederholungseinheit der Formel (4) gemäß Anspruch 6, welches das Umsetzen des Polyarylens gemäß Anspruch 6 mit einem Alkalimetallhalogenid oder einem quartären Ammoniumhalogenid, gefolgt von der Durchführung einer Säurebehandlung, umfasst.

20. Ein Verfahren zur Herstellung eines Polyarylens, bestehend aus der Wiederholungseinheit der Formel (7) gemäß Anspruch 14, welches das Hydrolysieren des Polyarylens gemäß Anspruch 4 in Gegenwart einer Säure oder Base umfasst.

21. Ein Verfahren zur Herstellung eines Polyarylens, bestehend aus der Wiederholungseinheit der Formel (7) gemäß Anspruch 14, welches das Umsetzen des Polyarylens gemäß Anspruch 4 mit einem Alkalimetallhalogenid oder einem quartären Ammoniumhalogenid, gefolgt von der Durchführung einer Säurebehandlung, umfasst.

22. Das Polyarylen gemäß Anspruch 1, wobei das Polyarylen die Wiederholungseinheit der Formel (2) gemäß Anspruch 1 enthält und wenigstens zwei Wiederholungseinheiten der Formel (2) gemäß Anspruch 1 folgen.

## Revendications

1. Polyarylène comprenant un motif répétitif représenté par la formule (2) : 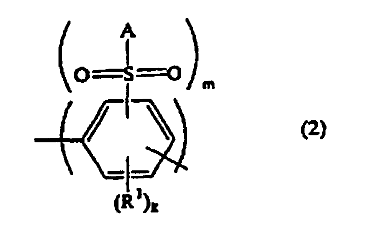 dans laquelle A représente un groupe alcoxy C3-C20, qui peut être substitué par au moins un groupe choisi dans le groupe constitué par un atome de fluor, un groupe alcoxy C1-C20, un groupe aryle C6-C20, un groupe aryloxy C6-C20, un groupe acyle C2-C20 et un groupe cyano,
R¹ représente un atome d'hydrogène, un atome de fluor, un groupe alkyle C1-C20, un groupe alcoxy C1-C20, un groupe aryle C6-C20, un groupe aryloxy C6-C20, un groupe acyle C2-C20 ou un groupe cyano, et le groupe alkyle C1-C20, le groupe alcoxy Cl-C20, le groupe aryle C6-C20, le groupe aryloxy C6-C20, et le groupe acyle C2-C20 peuvent être substitués par au moins un substituant choisi dans le groupe constitué par un atome de fluor, un groupe cyano, un groupe alcoxy C1-C20, un groupe aryle C6-C20 et un groupe aryloxy C6-C20, et quand il existe de multiples R¹, les R¹ peuvent être des groupes identiques ou des groupes différents, et les deux R¹ voisins peuvent être liés pour former un cycle,
X¹ représente un atome de chlore, un atome de brome ou un atome d'iode, m représente 1 ou 2, et k représente 4-m.

2. Polyarylène selon la revendication 1, dans lequel m est 1.

3. Polyarylène selon la revendication 1, dans lequel R¹ est un atome d'hydrogène.

4. Polyarylène constitué par un motif répétitif représenté par la formule (2) selon la revendication 1.

5. Polyarylène selon la revendication 1, dans lequel le polyarylène comprend un motif répétitif représenté par la formule (2) selon la revendication 1 et un segment représenté par la formule (3) : 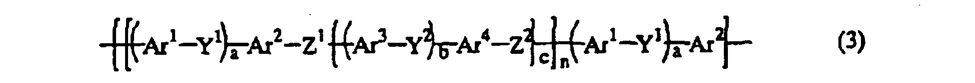 dans laquelle a, b et c sont identiques ou différents, et chacun représente 0 ou 1, et n représente un nombre entier de 5 ou plus, Ar¹, Ar², Ar³ et Ar⁴ sont identiques ou différents, et chacun représente un groupe aromatique divalent, et le groupe aromatique divalent peut être substitué par au moins un substituant choisi dans le groupe constitué par
un groupe alkyle C1-C20 qui peut être substitué par au moins un substituant choisi dans le groupe constitué par un atome de fluor, un groupe cyano, un groupe alcoxy C1-C20, un groupe aryle C6-C20 et un groupe aryloxy C6-C20 ;
un groupe alcoxy C1-C20 qui peut être substitué par au moins un substituant choisi dans le groupe constitué par un atome de fluor, un groupe cyano, un groupe alcoxy C1-C20, un groupe aryle C6-C20 et un groupe aryloxy C6-C20 ;
un groupe aryle C6-C20 qui peut être substitué par au moins un substituant choisi dans le groupe constitué par un atome de fluor, un groupe cyano, un groupe alcoxy C1-C20 et un groupe aryloxy C6-C20 ;
un groupe aryloxy C6-C20 qui peut être substitué par au moins un substituant choisi dans le groupe constitué par un atome de fluor, un groupe cyano, un groupe alcoxy C1-C20 et un groupe aryloxy C6-C20 ; et
un groupe acyle C2-C20 qui peut être substitué par au moins un substituant choisi dans le groupe constitué par un atome de fluor, un groupe cyano, un groupe alcoxy C1-C20, un groupe aryle C6-C20 et un groupe aryloxy C6-C20 ;
Y¹ et Y² sont identiques ou différents, et chacun représente une liaison simple, un groupe carbonyle, un groupe sulfonyle, un groupe 2,2-isopropylidène, un groupe 2,2-hexafluoroisopropylidène ou un groupe fluorène-9,9-diyle, et
Z¹ et Z² sont identiques ou différents, et chacun représente un atome d'oxygène ou un atome de soufre.

6. Polyarylène selon la revendication 1, dans lequel le polyarylène comprend un motif répétitif représenté par la formule (2) selon la revendication 1 et un motif répétitif représenté par la formule (4) :
-Ar⁵- (4)
dans laquelle Ar⁵ représente un groupe aromatique divalent, et le groupe aromatique divalent peut être substitué par au moins un substituant choisi dans le groupe constitué par
un groupe alkyle C1-C20 qui peut être substitué par au moins un substituant choisi dans le groupe constitué par un atome de fluor, un groupe cyano, un groupe alcoxy C1-C20, un groupe aryle C6-C20 et un groupe aryloxy C6-C20 ;
un groupe alcoxy C1-C20 qui peut être substitué par au moins un substituant choisi dans le groupe constitué par un atome de fluor, un groupe cyano, un groupe alcoxy C1-C20, un groupe aryle C6-C20 et un groupe aryloxy C6-C20 ;
un groupe aryle C6-C20 qui peut être substitué par au moins un substituant choisi dans le groupe constitué par un atome de fluor, un groupe cyano, un groupe alcoxy C1-C20 et un groupe aryloxy C6-C20 ;
un groupe aryloxy C6-C20 qui peut être substitué par au moins un substituant choisi dans le groupe constitué par un atome de fluor, un groupe cyano, un groupe alcoxy C1-C20 et un groupe aryloxy C6-C20 ; et
un groupe acyle C2-C20 qui peut être substitué par au moins un substituant choisi dans le groupe constitué par un atome de fluor, un groupe cyano, un groupe alcoxy C1-C20, un groupe aryle C6-C20 et un groupe aryloxy C6-C20.

7. Procédé de production d'un polyarylène comprenant le motif répétitif représenté par la formule (2) selon la revendication 1, qui comprend la polymérisation d'une composition de monomères comprenant le composé dihalogénobenzène représenté par la formule (1) : 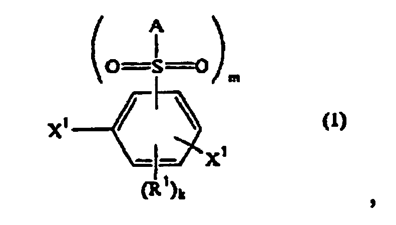 dans laquelle A, R¹, m et k ont les mêmes significations que celles définies dans la revendication 1, en présence d'un composé de nickel.

8. Procédé de production d'un polyarylène selon la revendication 7, dans lequel la composition de monomères comprend le composé dihalogénobenzène représenté par la formule (1) selon la revendication 7 et un composé représenté par la formule (5) : 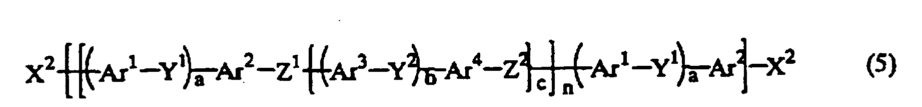 dans laquelle a, b, c, n, Ar¹, Ar², Ar³, Ar⁴, Y¹, Y², Z¹ et Z² ont les mêmes significations que celles définies dans la revendication 5, et X² représente un atome de chlore, de brome ou d'iode.

9. Procédé de production d'un polyarylène selon la revendication 7, dans lequel la composition de monomères comprend le composé dihalogénobenzène représenté par la formule (1) selon la revendication 7 et un composé représenté par la formule (6) :
X³-Ar⁵-X³ (6)
dans laquelle Ar⁵ a la même signification que celle définie dans la revendication 6, et X³ représente un atome de chlore, de brome ou d'iode.

10. Procédé de production d'un polyarylène selon la revendication 7, dans lequel le polyarylène est constitué par des motifs répétitifs représentés par la formule (2) selon la revendication 1, ledit procédé comprenant la polymérisation du composé dihalogénobenzène représenté par la formule (1) selon la revendication 7 uniquement.

11. Procédé de production d'un polyarylène selon l'une quelconque des revendications 7 à 10, dans lequel le composé de nickel est un bis(cyclooctadiène)nickel(0) et la polymérisation est mise en oeuvre en présence d'un ligand bidenté azoté.

12. Procédé de production d'un polyarylène selon l'une quelconque des revendications 7 à 10, dans lequel le composé de nickel est un bis(cyclooctadiène)nickel(0) et la polymérisation est mise en oeuvre en présence d'un ligand bidenté azoté et de zinc.

13. Procédé de production d'un polyarylène selon l'une quelconque des revendications 7 à 10, dans lequel le composé de nickel est un halogénure de nickel et la polymérisation est mise en oeuvre en présence d'un ligand bidenté azoté et de zinc.

14. Procédé de production d'un polyarylène comprenant un motif répétitif représenté par la formule (7) : 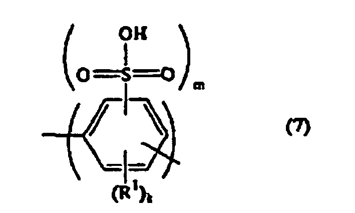 dans laquelle R¹, m et k ont les mêmes significations que celles définies dans la revendication 1, qui comprend l'hydrolyse du polyarylène selon la revendication 1 en présence d'un acide ou d'un alcali.

15. Procédé de production d'un polyarylène comprenant un motif répétitif représenté par la formule (7) selon la revendication 14, qui comprend la réaction du polyarylène selon la revendication 1 avec un halogénure de métal alcalin ou un halogénure d'ammonium quaternaire suivie par la mise en oeuvre d'un traitement à l'acide.

16. Procédé de production d'un polyarylène comprenant le motif répétitif représenté par la formule (7) selon la revendication 14 et le segment représenté par la formule (3) selon la revendication 5, qui comprend l'hydrolyse du polyarylène selon la revendication 5 en présence d'un acide ou d'un alcali.

17. Procédé de production d'un polyarylène comprenant un motif répétitif représenté par la formule (7) selon la revendication 14 et le segment représenté par la formule (3) selon la revendication 5, qui comprend la réaction du polyarylène selon la revendication 5 avec un halogénure de métal alcalin ou un halogénure d'ammonium quaternaire suivie par la mise en oeuvre d'un traitement à l'acide.

18. Procédé de production d'un polyarylène comprenant le motif répétitif représenté par la formule (7) selon la revendication 14 et le motif répétitif représenté par la formule (4) selon la revendication 6, qui comprend l'hydrolyse du polyarylène selon la revendication 6 en présence d'un acide ou d'un alcali.

19. Procédé de production d'un polyarylène comprenant un motif répétitif représenté par la formule (7) selon la revendication 14 et le motif répétitif représenté par la formule (4) selon la revendication 6, qui comprend la réaction du polyarylène selon la revendication 6 avec un halogénure de métal alcalin ou un halogénure d'ammonium quaternaire suivie par la mise en oeuvre d'un traitement à l'acide.

20. Procédé de production d'un polyarylène constitué du motif répétitif représenté par la formule (7) selon la revendication 14, qui comprend l'hydrolyse du polyarylène selon la revendication 4 en présence d'un acide ou d'un alcali.

21. Procédé de production d'un polyarylène constitué du motif répétitif représenté par la formule (7) selon la revendication 14, qui comprend la réaction du polyarylène selon la revendication 4 avec un halogénure de métal alcalin ou un halogénure d'ammonium quaternaire suivie par la mise en oeuvre d'un traitement à l'acide.

22. Polyarylène selon la revendication 1, dans lequel le polyarylène contient le motif répétitif représenté par la formule (2) selon la revendication 1 et au moins deux motifs répétitifs représentés par la formule (2) selon la revendication 1 à la suite.
